# EUROPEAN PATENT APPLICATION

(11) **EP 4 386 088 A1**
(43) Date of publication of application: **19.06.2024**
(21) Application number: 22213329.0
(22) Date of filing: 14.12.2022
(51) Int. Cl.: C12Q 1/6806, C12Q 1/6876

(54) **METHOD FOR THE ACCURATE QUANTIFICATION OF NON-CODING RNAS IN MINUTE QUANTITIES**

(71) Applicant: Johannes Gutenberg-Universität Mainz, 55122 Mainz (DE)
(72) Inventor: Kristen, Marco, 55128 Mainz (DE); Helm, Mark, 55128 Mainz (DE)
(74) Representative: Durm Patentanwälte PartG mbB

(57) **Abstract**

The present invention relates to methods for the quantification of one or more tRNA species in a tissue sample or cell sample derived from a specific organism.

## Description

The present invention relates to methods for the quantification of one or more tRNA species in a tissue sample or cell sample derived from a specific organism.

Transfer RNAs (tRNAs) are universally expressed small non-coding RNA molecules, bearing a distinct secondary structure and extensive levels of RNA modifications. tRNAs are crucial for protein biosynthesis in translating nucleic acid encoded information from messenger RNA (mRNA) to functional proteins. Alterations in the process of mRNA translation have been associated with various diseases in humans, comprising neurological diseases and cancer. Approaches to quantify these mRNAs in order to obtain gene expression profiles have recently proven insufficient on their own, missing impact of numerous post-transcriptional factors. At this point, ribosomes, the key player carrying out protein synthesis itself, are highly dependent on availability and abundance of tRNAs, thereby directly influencing speed and efficiency of protein synthesis. Considering the diverse pool of tRNAs, differing in structure, carried amino acid and especially their dynamic expression, an accurate and easily accessible method for tRNA pool quantification is required in biochemical and biomedical sciences.

Due to their high modification content, tRNAs are notoriously hard to quantify by deep sequencing and other methods. To account for the numerous biases resulting from concatenation of various enzymes involved, complicated workarounds and correction factors affect accuracy, sensitivity, and turnaround time.

Despite recent advances, the research field currently lacks an easily accessible and precise high-throughput method for fast and inexpensive characterization of tRNA pools from smallest tRNA quantities. Current methods based on gel electrophoresis offer acceptable accuracy in exchange for the significant effort and time consumption to prepare them, even for single tRNA subtypes. Methods utilizing the distinct sequence of every tRNA subtype to specifically target single tRNAs with an individually tailored nucleic acid counterpart (oligonucleotide), so-called tRNA microarrays, require tremendous preparational effort and are limited by their fluorescence-based detection. Due to this restrain, only few different samples can be analyzed for their full tRNA pool per microarray, thus limiting throughput-capability and therefore applicability for experiments with numerous conditions and replicates.

Based on advances in next generation sequencing (NGS) over the last decade, several methods tried to utilize the system's excellent throughput capability for tRNA quantification and characterization of very rare tRNA subtypes. However, this method is unable to directly analyze the tRNAs and thus requires numerous steps of preparative treatment. In a first step, RNA must be converted to DNA (reverse transcription), a step greatly impeded by structural peculiarities of tRNAs, such as the extensive secondary structure and a vast repertoire of over 160 modified ribonucleotides, each of them individually interfering with reverse transcription. Also, a so-called adapter ligation, used in most methods and based on enzymatic or chemical treatment, greatly varies in efficiency, introducing selection bias and increasing the required amount of tRNA for the method. The same applies to further preparative steps and the final polymerase chain reaction (PCR) which is required to introduce several sequences that are crucial for NGS. The sequence introduction in PCR is performed on widely differing sequences which display varying amplification efficiencies and can therefore introduce quantitative bias. Finally, frequently used reverse transcription quantitative PCR (RT-qPCR) struggles with the high abundance of modified ribonucleotides in tRNA, introducing severe quantitative bias.

Another important factor in this respect is the required input amount of tRNA which ranges around 1 to 2 µg for microarrays and gel-based approaches. This amount can be limiting in terms of preparative effort to acquire enough tRNA or biological availability, e.g. when analyzing minor tissue compartments, not yielding sufficient tRNA quantities due to their small size. Current NGS methods lowered the barrier in terms of input requirements, normally ranging between 50 and 500 ng per sample, an amount achievable in many preparations, however still at the limit of what is feasible when collecting rare or small cells/tissues and considering technical replicates.

Accordingly, the technical problem underlying the present invention is the provision of means for an easily accessible and precise high-throughput method for the fast and inexpensive characterization of tRNA pools in minute quantities.

The solution to the above technical problem is achieved by the embodiments characterized in the claims.

In particular, in a first aspect, the present invention relates to a method for the quantification of one or more tRNA species in a tissue sample or cell sample derived from a specific organism, comprising the steps of:
(a) providing a tissue sample or cell sample derived from a specific organism;
(b) extracting total RNA from said tissue sample or cell sample;
(c) adding one or a mixture of more than one tRNA-complementary DNA oligonucleotide(s) to the total RNA obtained in step (b),
   wherein each of said tRNA-complementary DNA oligonucleotides contains a nucleotide sequence that specifically hybridizes to one specific tRNA of the organism from which the tissue or cell sample is derived, or to one specific tRNA family of said organism;
(d) allowing for hybridization of the tRNA-complementary DNA oligonucleotides added in step (c) to the tRNAs, thereby forming tRNA-tRNA-complementary DNA oligonucleotide heteroduplexes;
(e) performing native polyacrylamide gel electrophoresis with the mixture obtained in step (c) after hybridization in step (d), thereby separating tRNA-tRNA-complementary DNA oligonucleotide heteroduplexes, non-hybridized tRNA-complementary DNA oligonucleotides, and other RNA species from each other;
(f) purifying the tRNA-tRNA-complementary DNA oligonucleotide heteroduplexes from the native polyacrylamide gel;
(g) performing an index polymerase chain reaction (index PCR) on the tRNA-tRNA-complementary DNA oligonucleotide heteroduplexes obtained in step (f) using suitable index primers, thereby separating the tRNA-complementary DNA oligonucleotides from the tRNAs;
(h) purifying the PCR product obtained in step (g);
(i) subjecting the purified PCR product obtained in step (h) to NGS; and
(j) counting the number of times each tRNA-complementary DNA oligonucleotide sequence is found in the sequencing data obtained in step (i), wherein said number directly corresponds to the abundance of the respective tRNA species in the cell sample or tissue sample.

As used herein, the term "quantification" relates to the determination of the relative amounts of the one or more tRNA species in the tissue sample or cell sample by way of the methods of the present invention, or to the determination of the absolute amounts of the one or more tRNA species in the tissue sample or cell sample by way of the methods of the present invention, in case a standard of known tRNA concentration is added.

In the methods of the present invention, one or more tRNA species in a tissue sample or cell sample can be quantified, e.g. one tRNA species of particular interest, two or more tRNA species of particular interest, or, preferably, all tRNA species contained or suspected of being contained in said sample. In preferred embodiments, all known tRNA species for a given specific organism can be quantified by way of the methods of the present invention. tRNA species that can be quantified in the present invention are cytosolic tRNA species, mitochondrial tRNA species, or both cytosolic and mitochondrial tRNA species.

In this context, the term "tRNA species" as used herein refers to either (i) one specific tRNA that is characterized by a unique nucleotide sequence to which the tRNA-complementary DNA oligonucleotides used in the present invention specifically hybridize, said one specific tRNA further having a unique anticodon and carrying a unique amino acid, or (ii) one specific tRNA family comprising tRNAs that are characterized by a nucleotide sequence to which the tRNA-complementary DNA oligonucleotides used in the present invention specifically hybridize, but which differ from each other in 4, 3, 2, or 1, preferably in 1, nucleotide position(s), wherein said nucleotide position can be within the sequence to which the tRNA-complementary DNA oligonucleotides hybridize or outside of said sequence, wherein said tRNAs further have a common unique anticodon and carry a common unique amino acid (i.e., the members of the tRNA family are isoacceptors with respect to the amino acid, and isodecoders with respect to the anticodon).

As used herein, the term "tissue sample or cell sample" is not particularly limited and refers to any sample containing, or suspected of containing, a sufficient quantity of any tissue or tissues derived from a specific organism, and any sample containing a sufficient quantity of any type of cell or types of cells derived from a specific organism, including cells from unicellular organisms, e.g. eukaryotic unicellular organisms, such as protozoa (e.g. *Leishmania sp.)* or yeasts, as well prokaryotic organisms, such as bacteria (e.g. *Mycoplasma sp.)* or archaebacteria. Further, as used herein, the term "tissue sample or cell sample" further includes samples containing, or suspected of containing, viruses containing tRNA. By way of example, samples that might be of interest for analysis according to the methods of the present invention include samples that are suspected of being contaminated by one or more tRNA-containing species, e.g. cell culture samples suspected of having bacterial (e.g. mycobacterial) contaminations, environmental samples such as soil or water samples, samples of tissues suspected of being infected by a pathogenic agent, and the like.

In this context, the term "sufficient quantity" refers to a quantity that is large enough so that tRNAs contained in the sample can be quantified by way of the methods of the present invention. In this context, in preferred embodiments, the tissue sample or cell sample contains 50 ng or less total tRNA. More preferably, the tissue sample or cell sample contains 50 ng or less and 5 ng or more total tRNA, e.g. 5, 10, 15, 20, 25, 30, 35, 40, 45, or 50 ng total tRNA, or any amount of total tRNA in a range taking these values as lower and upper limits. In related embodiments, the tissue sample or cell sample contains 2 pmol or less total tRNA.

More preferably, the tissue sample or cell sample contains 2 pmol or less and 0.2 pmol or more total tRNA, e.g. 0.2, 0.4, 0.6, 0.8, 1.0, 1.2, 1.4, 1.6, 1.8, or 2.0 pmol total tRNA, or any amount of total tRNA in a range taking these values as lower and upper limits.

Further, as used herein, the term "derived from a specific organism" refers to the fact that typically, the tissue sample or cell sample contains a tissue or multiple tissues, or a type of cell or multiple types of cells, that are all taken from one or more individual organisms of one particular known species. However, in other embodiments, the tissue sample or cell sample contains a tissue or multiple tissues, or a type of cell or multiple types of cells, that are taken from individual organisms of more than one particular species, wherein at least one species for which the quantification of tRNAs is to be performed is known, or wherein all species for which the quantification of tRNAs is to be performed are known.

Accordingly, the term "tissue sample or cell sample derived from a specific organism" covers the following situations: (i) the tissue sample or cell sample contains one or more types of tissue and/or one or more types of cells, wherein all types of tissue and all types of cells are taken from one or more individual organisms of one known species; (ii) the tissue sample or cell sample contains one or more types of tissue and/or one or more types of cells, wherein the types of tissue and types of cells are taken from individual organisms of more than one species, wherein the quantification of tRNAs is to be performed for one known species of said more than one species; and (iii) the tissue sample or cell sample contains one or more types of tissue and/or one or more types of cells, wherein the types of tissue and types of cells are taken from individual organisms of more than one species, wherein the quantification of tRNAs is to be performed for more than one known species of said more than one species. In this context, samples that might be of interest for analysis according to the methods of the present invention include environmental samples or tissue samples in which tRNAs of multiple species of interest can be analyzed in parallel, thereby providing e.g. a microbiome analysis of the respective sample.

In this context, suitable species the tRNA of which can be quantified in the methods of the present invention are not particularly limited and include any prokaryotic or eukaryotic, unicellular or multicellular organisms, as well as viruses containing tRNA, e.g. eukaryotic unicellular organisms, such as protozoa (e.g. *Leishmania sp.)* or yeasts, as well prokaryotic organisms, such as bacteria (e.g. *Mycoplasma sp.)* or archaebacteria. In preferred embodiments, the tissue sample or cell sample is derived from *Homo sapiens, Mus musculus, Saccharomyces cerevisiae,* or *Escherichia coli.* In related specific embodiments, (i) the tissue sample or cell sample is derived from *Homo sapiens,* and the one or more than one tRNA-complementary DNA oligonucleotide(s) are selected from the group consisting of oligonucleotides having the nucleotide sequences of SEQ ID NOs: 126 to 191; (ii) the tissue sample or cell sample is derived from *Mus musculus,* and the one or more than one tRNA-complementary DNA oligonucleotide(s) are selected from the group consisting of oligonucleotides having the nucleotide sequences of SEQ ID NOs: 192 to 258; (iii) the cell sample is derived from *Saccharomyces cerevisiae,* and the one or more than one tRNA-complementary DNA oligonucleotide(s) are selected from the group consisting of oligonucleotides having the nucleotide sequences of SEQ ID NOs: 64 to 125; or (iv) the cell sample is derived from *Escherichia coli,* and the one or more than one tRNA-complementary DNA oligonucleotide(s) are selected from the group consisting of oligonucleotides having the nucleotide sequences of SEQ ID NOs: 24 to 63.

In step (a) of the methods of the present invention, the tissue sample or cell sample is provided. This expressly excludes any steps of actually obtaining or taking said tissue sample or cell sample from the respective organism. Accordingly, the methods of the present invention are preferably *in vitro* methods, i.e., methods that are not performed on the human or animal body.

In step (b) of the methods of the present invention, total RNA is extracted from the tissue sample or cells sample. In this context, the term "total RNA" encompasses transfer RNA (tRNA), messenger RNA (mRNA), ribosomal RNA (rRNA), and other RNA species known in the art. Means for extracting total RNA from a given tissue sample or cell sample are not particularly limited and are known in the art. Such means include e.g. methods based on organic extraction, using for example phenol-guanidine isothiocyanate (GITC)-based solutions, methods based on silica-membrane based spin column technology, methods based on paramagnetic particle technology, and hot-phenol RNA extraction methods. Specific means for extracting total RNA from a given tissue sample or cell sample are as described hereinafter (cf. section "Material and methods", *infra).*

In specific embodiments, the methods of the present invention can further comprise a step of (b') isolating tRNA from the total RNA after step (b) and prior to step (c), wherein in step (c), the one or a mixture of more than one tRNA-complementary DNA oligonucleotide(s) are added to the tRNA isolated in step (b'), instead of to the total RNA obtained in step (b). Means for isolating tRNA from a total RNA preparation are not particularly limited and are known in the art. Such means include the separation on a polyacrylamide gel electrophoresis (PAGE) by size of the RNA, as well as chromatographic methods such as ion exchange chromatography or specialized HPLC-methods known in the art.

In step (c) of the methods of the present invention, one or a mixture of more than one tRNA-complementary DNA oligonucleotide(s) is/are added to the total RNA obtained in step (b) (or isolated tRNA obtained in step (b')). Specifically, for each of the tRNA species that should be quantified in the tissue sample or cell sample, one specific tRNA-complementary DNA oligonucleotide is added. In this context, the term "tRNA-complementary DNA oligonucleotide" (also referred to herein as "cDNA Oligo for Quantification (cDOQ)" or "cDNA") refers to DNA oligonucleotides that contain a nucleotide sequence that specifically hybridizes, preferably under stringent conditions, to one and only one specific tRNA that should be quantified, or to one and only one specific tRNA family that should be quantified.

As used herein the terms "hybridizes" and "specifically hybridizes" refers to the process wherein a nucleotide sequence of the DNA oligonucleotide anneals to a complementary nucleotide sequence of the tRNA by way of canonical Watson-Crick hydrogen bonding, i.e., canonical Watson-Crick base pairing. Further, the term "stringent conditions" as used herein refers to conditions of high temperature during hybridization and low salt concentrations in the hybridization buffer that favor or only allow for hybridization of perfectly complementary nucleotide sequences, or allow for at most 10% of bases not matching. Suitable stringent hybridization conditions are known in the art and include e.g. the following: 30 mM HEPES pH 7.5 , 100 mM potassium acetate; temperature for initial denaturation (strands are separated) at 94°C for 2 mins, afterwards gradual cooling down to 25°C within 25 minutes (strands anneal while cooling, hybrids form).

In preferred embodiments, the hybridization region of the tRNA-complementary DNA oligonucleotides comprises about 40 nucleotides, e.g. 35 to 45, 36 to 44, 37 to 43, 38 to 43, or 39 to 41 nucleotides (nt). Further, the hybridization region of the tRNA-complementary DNA oligonucleotides can preferably hybridize to the 3'-end of the tRNAs, or the 5'-end of the tRNA. However, embodiments in which the tRNA-complementary DNA oligonucleotides can hybridize to any internal region of the tRNA are also contemplated herein.

In specific embodiments, the tRNA-complementary DNA oligonucleotides added in step (c) contain nucleotide sequences that are required for further sequencing on a next generation sequencing (NGS) platform up- and downstream of said nucleotide sequence that specifically hybridizes to one specific tRNA of the organism from which the tissue or cell sample is derived. Such sequences include sites that are designed to fit the standard primers which are provided for NGS techniques, e.g. in NGS sequencing kits. These sequences are needed for (i) annealing of index primers in polymerase chain reaction and (ii) binding of primers in the NGS sequencing process which allows sequence determination itself. Otherwise, suitable sequences in this respect are not particularly limited, are known in the art and/or can be designed by the person skilled in the art.

Further, in preferred embodiments, the tRNA-complementary DNA oligonucleotides contain a fluorescent tag, e.g. at their 3'- or 5'-end, preferably at their 5'-end. Suitable fluorescent tags, as well as means for coupling such tags to oligonucleotides, are not particularly limited and are known in the art. Preferably, the fluorescent tag is selected from the group consisting of 6-carboxyfluorescein and cyanine-5 (Cy5). Alternatively, the tRNA-complementary DNA oligonucleotides can contain any other types of tag that might be helpful in downstream processing steps, e.g. biotin, crosslinkers or moieties that can be derivatized via click-chemistry, wherein said moieties in turn can be derivatized with a fluorescent tag, biotin or crosslinker.

Preferably, in step (c) of the methods of the present invention, a mixture of tRNA-complementary oligonucleotides for each tRNA species of the specific organism is added to the total RNA obtained in step (b). Further, in case a mixture of more than one tRNA-complementary DNA oligonucleotides is added to the total RNA obtained in step (b), the different tRNA-complementary DNA oligonucleotides are present in said mixture e.g. in equimolar amounts, or in amounts corresponding to the expected distribution of tRNAs in the sample.

In step (d) of the methods of the present invention, hybridization of the tRNA-complementary DNA oligonucleotides added in step (c) to the tRNAs is allowed for. Preferably, this step is carried out under stringent conditions as defined above. Typically, this step can require denaturing of the solution containing the tRNAs and the tRNA-complementary DNA oligonucleotides, e.g. denaturation at about 94 °C for about 2 minutes, and subsequent gradual cooling of said solution, e.g. down to about 25 °C within about 25 minutes. Specific means for hybridization of the tRNA-complementary DNA oligonucleotides to the tRNAs are as described hereinafter (cf. section "Material and methods", *infra).*

In step (e) of the methods of the present invention, native polyacrylamide gel electrophoresis is performed with the mixture obtained in step (c) after hybridization in step (d), i.e., said mixture is subjected to native polyacrylamide gel electrophoresis after step (d). By way of this step, tRNA-tRNA-complementary DNA oligonucleotide heteroduplexes (also referred to herein as tRNA-tRNA-complementary DNA oligonucleotide hybrids, tRNA-oligo hybrids, or tRNA-cDOQ hybrids), non-hybridized tRNA-complementary DNA oligonucleotides, and other RNA species are separated from each other, e.g. based on their size and/or shape. Means for performing a respective native polyacrylamide gel electrophoresis are not particularly limited and are known in the art. Specific means for performing a respective native polyacrylamide gel electrophoresis are as described hereinafter (cf. section "Material and methods", *infra).*

In step (f) of the methods of the present invention, the tRNA-tRNA-complementary DNA oligonucleotide heteroduplexes are purified from the native polyacrylamide gel. The respective band or bands can e.g. be identified via a fluorescent tag that is attached to the tRNA-complementary DNA oligonucleotides, aiding visualization and thus identification of hybrid bands. Means for purifying the tRNA-tRNA-complementary DNA oligonucleotide heteroduplexes from the native polyacrylamide gel are not particularly limited and are known in the art. Such means include e.g. excision of the respective band(s) from the native polyacrylamide gel and elution of the tRNA-tRNA-complementary DNA oligonucleotide heteroduplexes from the excised gel fragment(s). Specific means for purifying the tRNA-tRNA-complementary DNA oligonucleotide heteroduplexes from the native polyacrylamide gel are as described hereinafter (cf. section "Material and methods", *infra).*

In step (g) of the methods of the present invention, an index polymerase chain reaction (index PCR; barcoding PCR) on the tRNA-tRNA-complementary DNA oligonucleotide heteroduplexes obtained in step (f) is performed using suitable index primers, thereby separating the tRNA-complementary DNA oligonucleotides from the tRNAs. Means for performing a respective PCR are not particularly limited and are known in the art. Specific means for performing a respective PCR are as described hereinafter (cf. section "Material and methods", *infra).*

In preferred embodiments, this PCR is performed for 2 to 6 PCR cycles, e.g. for 2, 3, 4, 5, or 6 cycles, preferably for 6 cycles. Of note, the index PCR in step (g) targets only the tRNA-complementary DNA oligonucleotides. Preferably, during the index PCR in step (g), further nucleotide sequences that are required for further sequencing on an NGS platform are added to said tRNA-complementary DNA oligonucleotides. Preferably, these further nucleotide sequences are primer binding sites.

Preferably, the index primers used in this step, examples for which are given in Table 2 below (SEQ ID NOs: 4 to 11, and 12 to 23) contain sequences for (i) binding to the flowcell of NGS sequencing systems, (ii) an index region (either i5 or i7 index/barcode) marked in bold in Table 2 below, and (iii) the region to bind the cDOQs in PCR, which at the same time are the regions NGS primers bind later on to perform the sequencing.

In step (h) of the methods of the present invention, the PCR product obtained in step (g) is purified, e.g. using a polyacrylamide gel or size exclusion column, or a kit for the purification of PCR products as known in the art. Respective means of purifying PCR products are not particularly limited and are known in the art. Specific means of purifying PCR products are as described hereinafter (cf. section "Material and methods", *infra).*

In step (i) of the methods of the present invention, the purified PCR product obtained in step (h) is subjected to NGS. In this respect, any NGS techniques and methods known in the art are advantageously amendable to the present invention. These include e.g. NGS systems using a so-called "sequencing by synthesis" approach that can be used for analysis of the retrieved PCR products. For this, all samples are mixed in an adequate, possibly equimolar ratio. Afterwards PCR products hybridize to a stationary phase, are then amplified and afterwards "sequencing" is performed. Hereby nucleobases with fluorescent dyes (different dyes for each of the 4 bases) are covalently attached to primers binding distinct sequences of the PCR product. Elongation can only be done by one nucleobase at a time, followed by a fluorescent image scan to gain information which base has been incorporated where. Afterwards, fluorescent dyes are removed, the nucleobases made eligible for further elongation and another cycle adding one fluorescent nucleobase begins. This is performed in numerous cycles on at least 3 regions of the PCR product, thus gaining the sequence of (i) the i5 barcode/index, (ii) the i7 barcode/index, with the combination of these allowing to specifically determine the sample this PCR product originated from and (iii) the 40nt sequence that identifies the specific tRNA that was bound previously in the hybridization process. Specific means for performing NGS in the context of the present invention are as described hereinafter (cf. section "Material and methods", *infra).*

Finally, in step (j) of the methods of the present invention, the number of times each tRNA-complementary DNA oligonucleotide sequence is found in the sequencing data obtained in step (i) is counted, wherein said number directly corresponds to the relative abundance of the respective tRNA species in the tissue sample or cell sample. More specifically, for each of the tRNA-complementary DNA oligonucleotides added in step (c) of the methods of the present invention, the number of times the specific sequence of each tRNA-complementary DNA oligonucleotides is found (e.g. the sequence that is complementary to the respective tRNA), is counted, wherein the number of counts directly correlates with the relative amount of the respective tRNA species in the tissue sample or cell sample. Using a calibration curve established using samples with known tRNA amounts, the absolute amount of tRNA in the tissue sample or cell sample can be determined for each tRNA species of interest. Counting of the sequences can be performed e.g. using open-source software tools known in the art, including e.g. custom scripts written in e.g. Python or R. Specific means for data analysis in this respect are as described hereinafter (cf. section "Material and methods", *infra).*

In preferred embodiments, the methods of the present invention do not comprise any further methods steps other than methods steps (a) to (j) as defined above. Further, the methods of the present invention preferably do not comprise any step of reverse transcription of tRNA.

In specific further aspects, the present invention relates to methods as defined above for the first aspect of the present invention, wherein the RNA to be quantified is not one or more tRNA species, but another RNA species, selected from the group consisting of small noncoding RNAs, e.g. microRNAs (miRNAs), small interfering RNAs (siRNAs), Piwi-interacting RNAs (piRNAs), small nucleolar RNAs (snoRNAs), small nuclear RNAs (snRNAs), extracellular RNAs (exRNAs), or small Cajal body-specific RNAs (scaRNAs).

In these aspects, all methods steps as defined for the methods according to the first aspect of the present invention equally apply. Further, all relevant limitations and definitions as provide for the above first aspect of the present invention equally apply.

Specifically, in these additional aspects, the tRNA-complementary DNA oligonucleotide(s) are small noncoding RNA-complementary DNA oligonucleotide(s), the tRNA-tRNA-complementary DNA oligonucleotide heteroduplexes are small noncoding RNA- small noncoding RNA-complementary DNA oligonucleotide heteroduplexes,

Thus, in these aspects, the present invention relates to a method for the quantification of one or more small noncoding RNA species in a tissue sample or cell sample derived from a specific organism, comprising the steps of:
(a) providing a tissue sample or cell sample derived from a specific organism;
(b) extracting total RNA from said tissue sample or cell sample;
(c) adding one or a mixture of more than one small noncoding RNA-complementary DNA oligonucleotide(s) to the total RNA obtained in step (b),
   wherein each of said small noncoding RNA-complementary DNA oligonucleotides contains a nucleotide sequence that specifically hybridizes to one specific small noncoding RNA of the organism from which the tissue or cell sample is derived, or to one specific small noncoding RNA family of said organism;
(d) allowing for hybridization of the small noncoding RNA-complementary DNA oligonucleotides added in step (c) to the small noncoding RNAs, thereby forming small noncoding RNA- small noncoding RNA-complementary DNA oligonucleotide heteroduplexes;
(e) performing native polyacrylamide gel electrophoresis with the mixture obtained in step (c) after hybridization in step (d), thereby separating small noncoding RNA- small noncoding RNA-complementary DNA oligonucleotide heteroduplexes, non-hybridized small noncoding RNA-complementary DNA oligonucleotides, and other RNA species from each other;
(f) purifying the small noncoding RNA- small noncoding RNA-complementary DNA oligonucleotide heteroduplexes from the native polyacrylamide gel;
(g) performing an index polymerase chain reaction (index PCR) on the small noncoding RNA- small noncoding RNA-complementary DNA oligonucleotide heteroduplexes obtained in step (f) using suitable index primers, thereby separating the small noncoding RNA-complementary DNA oligonucleotides from the tRNAs;
(h) purifying the PCR product obtained in step (g);
(i) subjecting the purified PCR product obtained in step (h) to NGS; and
(j) counting the number of times each small noncoding RNA-complementary DNA oligonucleotide sequence is found in the sequencing data obtained in step (i), wherein said number directly corresponds to the abundance of the respective small noncoding RNA species in the cell sample or tissue sample.

In these aspects, the small noncoding RNA species is preferably selected from the group consisting of microRNAs (miRNAs), small interfering RNAs (siRNAs), Piwi-interacting RNAs (piRNAs), small nucleolar RNAs (snoRNAs), small nuclear RNAs (snRNAs), extracellular RNAs (exRNAs), or small Cajal body-specific RNAs (scaRNAs).

As used herein, the term "comprising"/"comprises" expressly includes the terms "consisting essentially of"/"consists essentially of" and "consisting of"/"consists of", i.e., all of said terms are interchangeable with each other herein.

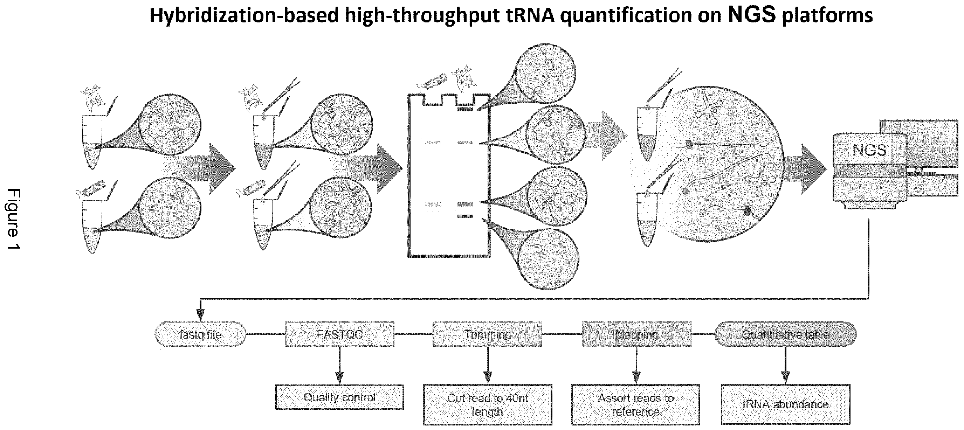

Further, as used herein, the term "about" refers to a modifier of the specified value of ± 10%, preferably ± 9%, ± 8%, ± 7%, ± 6%, ± 5%, ± 4%, ± 3%, ± 2%, ± 1%, ± 0.5%, or ± 0.1%. Thus, by way of example the term "about 100" as used herein can refer to ranges of 90 to 110, 91 to 109, 92 to 108, 93 to 107, 94 to 106, 95 to 105, 96 to 104, 97 to 103, 98 to 102, 99 to 101, 99.5 to 100.5, or 99.9 to 100.1. The methods for the quantification of tRNAs according to the present invention combine the excellent throughput capability of NGS with hybridization of DNA oligonucleotides to circumvent previously stated problems of NGS-based methods and achieve accurate tRNA pool characterization and quantification. The method is visualized in Figure 1.

Starting from total RNA (including tRNA, mRNA, rRNA and other RNA species) extracted from any kind of tissue or cells, a mixture of tRNA-complementary oligonucleotides is added to the total RNA. Each oligonucleotide bears a sequence that specifically binds (hybridizes) one tRNA species from a certain organism (e.g. human, mouse, etc.). For every tRNA subtype (isoacceptor) or tRNA family as defined above, one oligonucleotide is prepared, typically resulting in 30 to 50 per organism. These oligonucleotides carry specific sequences left and right (up- and downstream) of the complementary hybridization region that are required for further sequencing on an NGS platform. Additionally, a fluorescent tag is attached to every oligonucleotide to simplify visualization in later steps. After hybridization of the oligonucleotide mix with the respective RNA, the mixture is purified on a native polyacrylamide gel, thus separating hybrids, non-hybridized oligonucleotides and other RNAs physically, e,g, based on their size and/or shape. The purified hybrids are afterwards subjected to a PCR in which the hybrids separate again and NGS-required sequences are added to the DNA oligo. The number of PCR cycles can be adjusted depending on the tRNA input amount, with normally 2 to 6 cycles applied, thereby including required index primers while causing no substantial amplification, thereby avoiding significant quantification bias. Afterwards the PCR product is purified, e.g. via polyacrylamide gels or size exclusion columns. The retained PCR product can be directly subjected to NGS, being compatible with all major platforms. Finally, the bioinformatical analysis consists of counting the number of times each oligonucleotide sequence (read) was found in the retrieved sequencing data. Using one of many open-source tools to count the reads for each oligonucleotide, which is directly corresponding to the abundance (i.e., relative amount) of the respective tRNA species, the tRNA pool can be quantified without deeper bioinformatical knowledge and skills.

The essential innovation behind the methods of the present invention lies in the hybridization of complementary oligonucleotides already bearing sequences for later sequencing, thus transferring quantitative information from instable and complications-bearing RNA to a stable, easily producible, and processable DNA oligonucleotide. Therefore, quantification is not carried out on the RNA or an RNA-derived molecule but the DNA oligonucleotide. This advantageously simplifies library preparation and minimizes enzymatic steps down to only one, thereby greatly reducing possible bias-introduction.

Summarizing the previously described features, one advantage of the approach taken in the present invention includes a greatly reduced amount of input material. Due to several preparative features and the few steps required in sample preparation, the required RNA input can be reduced greatly, down to approximately 0.2 pmol (= 5 ng) for tRNA pools (e.g. 40 to 50 tRNAs in parallel) or even less for single tRNAs. A further decrease is possible with adjustments regarding fluorescent labels or PCR cycle number. The usage of oligonucleotides to hybridize RNAs and as template for the later PCR allows to omit the NGS-typical reverse transcription and adapter ligation, thus preventing potential bias introduced in a classical NGS approach for RNA quantification. The optimized PCR with minimal cycle number (e.g. 2 to 8 cycles or 2 to 6 cycles) greatly reduces the quantitative error compared to other PCR based preparations which normally include between 5 and 30 cycles of PCR. Furthermore, the methods of the present invention utilize the high-throughput capability of NGS, allowing quantification of numerous samples with a wide range of target RNAs in parallel. Finally, the bioinformatic analysis of the sequencing output is far simpler compared to classical NGS approaches due to the clearer data structure, only requiring a counting of predefined oligonucleotide sequences.

The methods of the present invention advantageously require only basic technological equipment for preparation which is commonly found in most biochemical laboratories. Sequencing of prepared samples is possible on many different NGS platforms, thus increasing freedom of choice and economic flexibility. The material costs for sample preparation are comparably low, as they only require one enzymatic step compared to multiple, partly very expensive enzymes, in normal NGS-based approaches. Finally, the preparation time is comparably short, requiring three days in total, considering overnight elution, 1 day without overnight elution and only 5 hours of hands-on time, thus allowing for parallel and time efficient laboratory work (cf. Table 1).

**Table 1: Time effort in sample preparation.**

| | **Total time for procedure** | **Hands-on time** | **Total time with gel extraction kit** | |
|---|---|---|---|---|
| **Day 1** | | | **Day 1** | |
| RNA extraction | 2h | 0.75 h | RNA extraction | 2h |
| Hybridization | 1 h | 0.5 h | Hybridization | 1 h |
| PAGE purification | 1.5 h | 0.5 h | PAGE purification | 1.5 h |
| Overnight elution | - | | Elution and purification | 1 h |
| **Day 2** | | | Index PCR | 1 h |
| Elution and purification | 4 h | 1 h | PAGE purification | 1.5 h |
| Index PCR | 1 h | 0.75 h | Elution and purification | 1 h |
| PAGE purification | 1.5 h | 0.5 h | | |
| Overnight elution | - | - | | |
| **Day 3** | | | | |
| Elution and purification | 4 h | 1 h | | |
| **Total** | **14 h** | **5 h** | **9h** | |

The work schedule from starting organism down to the sequencing ready library comprises three days in total, due to overnight incubation for classical PAGE gel elution in ammonium acetate solution. Utilization of DNA gel extraction kits omits overnight elution, thus allowing to prepare the libraries in just one day with approximately 5 hours hands-on time for 24 samples.

The present invention presents a hybrid approach that harnesses the advantages of hybridization-based and deep sequencing-based approaches. Quantitative information on the isoacceptor composition of a tRNA pool is transferred to a cDNA mixture in a single step procedure, thereby omitting all enzymatic conversions except for the subsequent barcoding PCR. As a result, a detailed tRNA composition matrix can be obtained from femtomolar amounts of total tRNA, corresponding to about one order of magnitude less material than in comparable approaches. The method is fast, low in cost, and its bioinformatic data workup very simple. These properties make the approach amenable to high-throughput investigations, as has been demonstrated by application to a collection of variegated biological questions, each answered with novel findings. These include tRNA pool quantification of polysome-bound tRNA, of tRNA modification knockout strains under stress conditions, and of Alzheimer patient's brain tissues. In summary, the method takes tRNA quantification to an advanced level of practical application to low input and high samples numbers.

In view of the numerous obstacles in tRNA quantification, a novel method was developed herein combining a cDNA hybridization technique with Next-Generation sequencing, thus overcoming reverse transcription (RT)-derived hinderances while achieving high-throughput capability. Complementary DNA oligonucleotides were designed targeting individual tRNA isoacceptors already containing adapter sequences required for final library preparation. The tRNA-cDNA hybridization transfers quantitative information to the cDNA template, thus omitting reverse transcription and any enzymatic step beside a low-cycle index PCR which takes place on the cDNA template. Following NGS, the limited pool of unmodified cDNA reference sequences allows for a greatly simplified bioinformatic analysis which is, in comparison to any other RNA sequencing approach, excessively simple and fast. Furthermore, preparational effort for 96 samples comprises only around 5 days with 15 to 20 hours of hands-on time and a total cost below 50$ per sample, considering consumables and outsourced NGS. Finally, the methods of the present invention allow for tRNA pool characterization with down to 5 ng of tRNA, lowering the RNA input requirements by one order of magnitude compared to current methods.

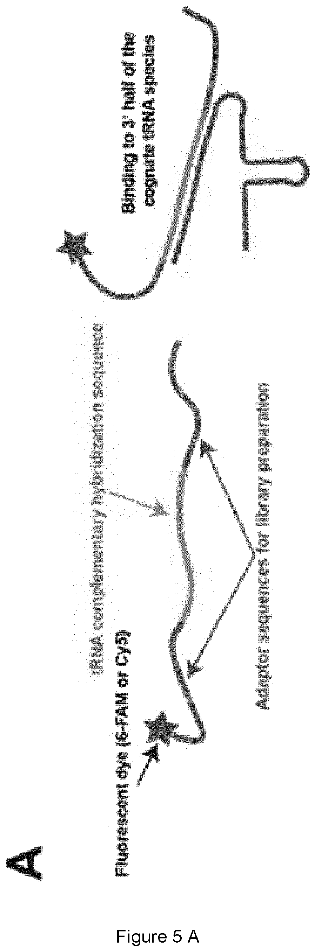

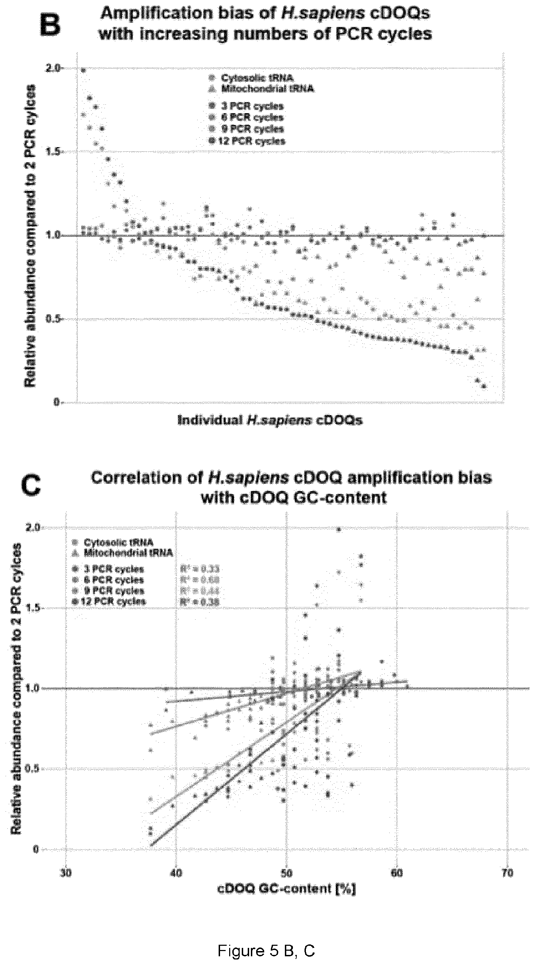

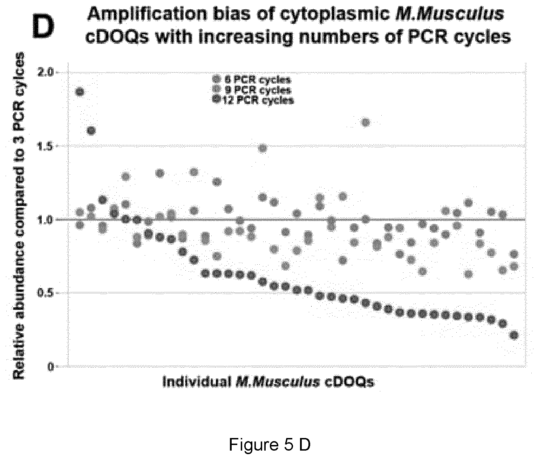

The figures show:
Figure 1:
   Overview of the method of the present invention.
   First, tRNA is extracted from any type of organism yielding total RNA varying in purity, dependent on the organism and extraction method. Retrieved total RNA is then mixed with an organism-specific set of hybridization DNA oligonucleotides that each target a specific tRNA and afterwards hybridized. In the next step, samples are run on a native polyacrylamide gel to separate unwanted RNA species and excess DNA from target tRNA-DNA-hybrids. Hereby, hybrids are selectively excised and eluted and afterwards subjected to index PCR. In this step the hybrids are separated again, and the DNA oligonucleotides are used as templates to introduce NGS-required sequences. Finally, all DNA oligos are transformed to full-length PCR products and can be subjected to NGS sequencing. Following sample preparation and sequencing, retrieved data can be conveniently analyzed due to the simple data structure only requiring counting of the predefined DNA oligonucleotide sequences.
Figure 2:
   Overview of the hybridization-based quantification method and major performance characteristics.
   (A) Starting from total RNA or isolated tRNA, an organism-specific mixture of hybridization oligos, called cDNA for Oligo Quantification (cDOQ) is added and hybridized to the tRNAs. Each cDOQ targets a specific tRNA isoacceptor or tRNA family isoacceptor and is fluorescently labeled with 6-carboxyfluorescein for cytosolic and Cy5 for mitochondrial tRNAs. Hybrids are separated from excess oligo and non-tRNA species on a native polyacrylamide gel. Excised hybrids are eluted, and afterwards subjected to a low-cycle index PCR of 2 to 6 cycles targeting only the DNA-oligos. The PCR-product is subjected to NGS sequencing, yielding a table of tRNA abundances after minor bioinformatic treatment. (B) Example of hybridization on a native PAGE gel scanned for tagged fluorescein and GelRed staining. tRNA-cDOQ hybrids (F1) are physically separated from unbound oligo on the gel, afterwards excised and subjected to Index PCR yielding full length product at 169 nt. Fraction F2 and F3 were analyzed in separate experiments (Fig. 5) to characterize potential bias. (C) Number of mapped reads yielded from samples prepared with decreasing amounts of tRNA displayed in pink compared to the correlation with a sample prepared from 100 ng tRNA. (D) Calibration and absolute quantification by standard addition of in vitro transcribed tRNA Thr ^{AGT} to 25 ng of total tRNA from HEK293 cells plotted against the measured Thr ^{AGT} abundance. The y-axis interception corresponds to the tRNA abundance of the sample.
Figure 3:
   Analysis of tRNA expression in different mammals focusing on mitochondria and Alzheimer's disease.
   (A) Expression profile of all 22 mitochondrial tRNAs in *H. sapiens* and *M*. *musculus* from frontal cortex tissue. tRNAs are labeled in one-letter amino acid code with anticodons listed for isoacceptors if applicable. (B) Composition of 44 cytosolic tRNAs in two human cell lines and human cortex tissue from male and female individuals, either bearing Alzheimer's disease (AD) or non-dement control (Ctr). The average abundance is visible in black with higher-than-average expression in green and lesser expression in blue. tRNAs are labeled in one-letter amino acid code with multiple anticodons listed when cDOQs target multiple isoacceptors at once. Anticodons are additionally numbered in cases where different isodecoders were targeted. (C) Fraction of mitochondrial tRNA of all tRNAs analyzed in human cortex tissue from male and female individuals, either bearing Alzheimer's disease (AD) or non-dement control (Ctr). Error bars indicate standard deviation and asterisk indicate a significant difference in two-sided t-test (**** p ≤ 0.0001) while n.s. indicates no significant difference. (D) Display of mitochondrial tRNA composition in human cell line and human frontal cortex tissue from male and female individuals, either bearing Alzheimer's disease (AD) or non-dement control (Ctr). Asterisks indicate a significant difference in two-sided t-test for the female AD group (** p ≤ 0.01, *** p ≤ 0.001).
Figure 4:
   Differing composition of cytosolic and polysomal tRNA pools of *E.coli* mutants under oxidative stress.
   (A) Expression profile of 40 tRNAs in either cytoplasmic (F0) or polysomal (F3) fraction of polysome preparations prepared from *E. coli* K12 wildtype and two mutant strains. tRNAs are labeled in one-letter amino acid code with anticodons listed for isoacceptors if applicable. (B) tRNA ^{fMet} abundance in different fractions (F0/F3) for different mutant strains. Error bars indicate standard deviation and asterisk indicate a significant difference in two-sided t-test (**p ≤ 0.01, *** p ≤ 0.001). (C) Relative abundance of tRNA ^{fMet} for different mutants in their respective polysomal fractions treated with different amounts of paraquat. Error bars indicate standard deviation and asterisk indicate a significant difference in two-sided t-test (*p ≤ 0.05, ** p ≤ 0.01) while n.s. indicates no significant difference.
Figure 5:
   cDOQ structure and PCR-derived bias in cDOQ quantities.
   (A) General structure of a cDNA oligo for quantification ("cDOQ") and its binding to the cognate tRNA. (B) Relative abundance of individual cDOQs in a mixture of *H. sapiens* cDOQs after different numbers of PCR cycles compared to the initial abundance measured after 2 PCR cycles. Data shape indicates cDOQs targeting cytosolic (circle) or mitochondrial (triangle) tRNAs. (C) Correlation of the cDOQ GC-content with the relative abundance of individual cDOQs in a mixture of *H*. *sapiens* cDOQs after different numbers of PCR cycles compared to the initial abundance measured after 2 PCR cycles. (D) Relative abundance of individual cDOQs in a mixture of *M. musculus* cDOQs after different numbers of PCR cycles compared to the initial abundance measured after 3 PCR cycles.
Figure 6:
   Investigation of oligo specificity, potential quantitative bias, and completeness of hybridization.
   (A) Percentage of reads mapped to either glycin tRNA isoacceptor after specifically targeting Gly ^{GCC} with the respective cDOQ. The isoacceptors display 6nt difference in the hybridization sequence (6nt = 15%). (B) Exemplary cDOQ specificity. (C) Ratio of RNAseq reads from different tRNA Gly isoacceptors before and after specific hybridization of tRNA Gly ^{GCC}. (D) In the process of hybridization, the noise derived from oligo secondary structures (F2) and its respective impact on hybrids (F1) and following quantification was investigated. Furthermore, completeness of hybridization was analyzed by RNAseq analysis of tRNA-height post-hybridization (F3). 12 fractions were prepared from four human total RNA samples with F2 running on the same hybridization gel. Following elution, 50 fmol of hybridization oligo targeting *E.coli* Phe ^{GAA} was added to all F1 and F2 fractions. Samples were subjected to PCR, sequenced, and afterwards analyzed for their *E.coli* Phe ^{GAA} oligo read proportion. From this, the initial oligo concentration could be calculated for each sample and used for impact analysis. (E) Correlation plots for all F1 and F2 samples comparing tRNA abundance in the respective sample with the average abundance across all four F1 fractions. (F) Comparison of mapped reads retrieved for hybridization oligos and tRNAs from RNAseq analysis of Fraction 3 (F3). (G) Mapping rate of uniquely aligned reads with the novel quantification approach across different species.

The present invention will be further illustrated by the following examples without being limited thereto.

### Examples

### Material and methods:

### Cell line samples

HEK293- (#ACC 305, DSMZ, Braunschweig, Germany), HeLa- (#ACC 057, DSMZ) and T98g- (#CRL-1690, ATCC, Manassas, VA, USA) cells were cultivated in Dulbecco's Modified Eagle's Medium (Thermo Fisher Scientific, Waltham, MA, USA) supplemented with 10 vol-% fetal bovine serum (Thermo Fisher Scientific) and 0.1% Primocin (InvivoGen, San Diego, CA, USA) at 37 °C and 5 vol-% CO₂. For separation or harvesting, cells were first washed with Dulbecco's Phosphate-Buffered Saline (Thermo Fisher Scientific) and then incubated with Trypsin-EDTA (Thermo Fisher Scientific) for five minutes under growing conditions. After detaching, cells were centrifuged for five minutes at 400 g, washed with phosphate buffer, and once again centrifuged. Finally, pellets were dissolved in TRI Reagent (Sigma-Aldrich, St. Louis, MO, USA) and stored at -20 °C.

### Murine tissue samples

Female C57BL/6J mice were used at an age of 16 weeks (for housing conditions and genotyping as known in the art. All experimental procedures were carried out in accordance with the European Communities Council Directive regarding care and use of animals for experimental procedures and was approved by local authorities (LUA-Rhineland-Palatinate).

### Human tissue samples

Human brain samples were provided by the rapid autopsy program of the Netherlands Brain Bank (NBB, Amsterdam, Netherlands) for high quality specimen from clinically well-documented and neuropathologically confirmed cases. Research on human specimens was performed according to the ethical declaration of the NBB. All cases of Alzheimer's disease were neuropathologically confirmed using the Consortium to Establish a Registry for Alzheimer's Disease (CERAD) criteria. The control specimen had no history or symptoms of neurologic or psychiatric disorders and showed no clinical symptoms of dementia. Tissue from the Gyrus frontalis superior 3+4 was used for all control and AD specimens.

### RNA isolation from cells or tissue

Total RNA was isolated from either cell pellets or dissected tissue by extraction with TRI Reagent (Sigma-Aldrich). The cell pellet or tissue was suspended in 2 mL TRI Reagent and thoroughly mixed. Following addition of 400 µL chloroform (Carl Roth, Karlsruhe, Germany) and thorough mixing, samples were centrifuged at 18,000 g for 15 minutes, the aqueous phase transferred to a new tube and 1 mL 2-propanol (Carl Roth), as well as 1 µL Glycogen (Thermo Fisher Scientific), were added. Following another round of centrifugation at 18,000 g for 15 minutes, supernatant was removed and 1 mL ethanol (75%) (Thermo Fisher Scientific) was added for a final centrifugation step. Finally, supernatant was carefully removed, the RNA pellet briefly airdried and resuspended in RNase free water.

The required amount of TRI Reagent varied depending on the number of cells or tissue size used, with respective changes to the other Reagents that were added.

### Polysome profiling and tRNA isolation

The *E. coli* wildtype strain Keio parent (BW25113) and the knockout strains of DusA and DusB were purchased from the *E. coli* Keio knockout collection (GE Healthcare (DharmaconTM, England). *E. coli* cultures were grown in 100 mL LB medium (Carl Roth) at 37 °C and 190 rpm until an optical density of 0.4 at 600 nm. Paraquat dichloride hydrate (Sigma-Aldrich) was added to final concentrations of 0, 0.1 and 0.3 mM and bacterial growth was continued until an OD₆₀₀ of 0.7. Chloramphenicol (100 µg/mL, Carl Roth) was added to the culture which was incubated for 3 min and then harvested by centrifugation (10 min, 10,000 g, 4 °C). The pelleted cells were resuspended in buffer (100 mM NH₄Cl (Merck, Darmstadt, Germany), 10 mM MgCl₂ (Carl Roth), 20 mM Tris, pH 7.5 (Carl Roth)), lysozyme (Carl Roth) was added and freeze-thaw cycles in liquid nitrogen were performed. Lysis was completed by adding 10% deoxycholate (Sigma-Aldrich) and cell wall debris was removed by centrifugation (12,000 g, 10 min, 4 °C). Cell lysate was loaded on top of sucrose gradients from 5 to 40% using Biocomp (BioComp, Fredericton, Canada) gradient station model 108 (settings: time 1.23 min, angle 81.5 °, speed 21 rpm). Gradients were ultracentrifuged (150,000 g, 4 °C, 2.5 h, Beckman Optima MAX-XP Ultracentrifuge, SW40 Ti rotor from Beckman Coulter, CA, USA) and fractionated collecting free RNA fraction (F0) and polysomal fraction (F3). Total RNA was extracted using TRI Reagent (Sigma-Aldrich) and separated subsequently on a 10% denaturing PAGE gel stained with GelRed (Biotium/BIOTREND, Köln, Germany). tRNA bands were excised from the gel after visualization of RNA bands using Typhoon 9400 (excitation wavelength of 532 nm, Amersham Bioscience/GE Healthcare, Chicago, IL, USA) and mashed with a scalpel. tRNA was extracted from the gel adding 300 µL 0.5 M ammonium acetate (Merck) before overnight incubation at 25 °C and 750 rpm. After filtration through NanoSep (VWR, Darmstadt, Germany) centrifugal filters, RNA was precipitated using three volumes of 100% ethanol (Carl Roth).

### cDOQ design

cDNA oligonucleotides for quantification (cDOQs) were designed from tRNA references retrieved from tRNAdb, MODOMICS, and gtRNAdb. In addition to sequences from tRNAdb and MODOMICS, transfer RNAs predicted from genomic loci were compiled to non-redundant references utilizing a R-based construction pipeline known in the art. References constructed from this pipeline were compared with tRNAdb and MODOMICS to consider RNA modifications. Next, the last 40 nucleotides towards the tRNA 3' end were used to generate the reverse-complement of this sequence and merged with the respective P5 (AGACGTGTGCTCTTCCGATCT; SEQ ID NO: 1) and P3 (GATCGTCGGACTGTAGAACTCTGAAC; SEQ ID NO: 2) sequence on the 5' and 3' end. The full-length constructs of 87 nucleotides were finalized with either a 6-FAM (cytosolic tRNAs) or Cyanine-5 (mitochondrial tRNAs) attached to the 5' end. All cDOQs were ordered and synthesized at biomers.net GmbH (Ulm, Germany) or Integrated DNA Technologies (Coralville, IA, USA).

### PCR and sequencing primer design

For indexing PCR 12 i7 and 8 i5 primers were designed to allow multiplexing up to 96 samples. To reach full compatibility with Illumina platforms, a custom primer for the i5 index read on Illumina NextSeq platforms was designed and utilized on the sequencing platform. All Primers were ordered and synthesized at biomers.net GmbH.

### cDOQ-tRNA hybridization and hybrid purification

Starting from 50-200 ng of total RNA or 5-50 ng of total tRNA, 5× hybridization buffer (150 mM HEPES pH 7.5 (Carl Roth), 500 mM potassium acetate (Carl Roth)) was added. Following addition of an equimolar mixture of cDOQs for the selected targets (e.g. a single tRNA or 44 cytosolic + 22 mitochondrial tRNAs in human), the solution was denatured at 94 °C for 2 minutes and afterwards gradually cooled down to 25 °C within 25 minutes. The sample was then analyzed on a 10% native Polyacrylamide-gel. Bands of interest (e.g. cDOQ:tRNA hybrid) were excised and eluted via overnight elution in 0.5 M ammonium acetate (Merck) at 750 rpm and 15°C. Afterwards, gel particles were removed with Nanosep 0.45 µM spin columns (VWR), RNA in the aqueous phase ethanol precipitated and resuspended in RNase-free water.

### Indexing PCR and next generation sequencing

Purified cDOQ:tRNA hybrids were subjected to index PCR, adding individual i5 and i7 primer combinations to each sample. Reaction was carried out with Taq-Polymerase (NEB, Frankfurt am Main, Germany) and the reaction mixture purified on a denaturing PAGE, excising the product band around 169 nt length. Following overnight elution, ethanol precipitation and resuspension as described previously, samples were sequenced on an Illumina NextSeq 2000 or MiSeq platform (Illumina, San Diego, CA, USA). For sequencing on the NextSeq 2000 platform, a custom i5 index read primer was used.

### RNAseq

Library preparation of tRNA fractions was based on the NEBnext Small RNA Library Prep Set for Illumina (NEB) with custom adaptations and is briefly described below. First, tRNA was dephosphorylated with Antarctic Phosphatase (NEB) and afterwards phosphorylated with Polynucleotide Kinase (NEB). Following purification via RNeasy MinElute Cleanup kit (Qiagen, Hilden, Germany), a preadenylated 3' adaptor was ligated, and the reverse transcription primer hybridized to minimize adaptor-dimer formation. Next, the 5' adaptor was ligated, and reverse transcription was performed using SuperScript IV (Thermo Fisher Scientific) for its high sequence fidelity and ability to process sites comprising RNA modifications. Finally, polymerase chain reaction was performed using custom i5 and i7 indexes (Table 2) and the PCR product purified via gel excision on a denaturing polyacrylamide gel. Full length product was then subjected to sequencing on an Illumina NextSeq 2000 platform using a custom i5 index read primer.

**Table 2: Custom read and indexing primers**

| **Primer** | **Sequence** | **SEQ ID NO:** |
|---|---|---|
| i5 index read | 5'-GATCGTCGGACTGTAGAACTCTGAAC-3' | 3 |
| i 501 | 5'-AATGATACGGCGACCACCGAGATCTACAC**TATAGCCT**GTTCAGAGTTCTACAGTCCGACGATC-3' | 4 |
| i 502 | 5'-AATGATACGGCGACCACCGAGATCTACAC**ATAGAGGC**GTTCAGAGTTCTACAGTCCGACGATC-3' | 5 |
| i 503 | 5'-AATGATACGGCGACCACCGAGATCTACAC**CCTATCCT**GTTCAGAGTTCTACAGTCCGACGATC-3' | 6 |
| i 504 | 5'-AATGATACGGCGACCACCGAGATCTACAC**GGCTCTGA**GTTCAGAGTTCTACAGTCCGACGATC-3' | 7 |
| i 505 | 5'-AATGATACGGCGACCACCGAGATCTACAC**AGGCGAAG**GTTCAGAGTTCTACAGTCCGACGATC-3' | 8 |
| i 506 | 5'-AATGATACGGCGACCACCGAGATCTACAC**TAATCTTA**GTTCAGAGTTCTACAGTCCGACGATC-3' | 9 |
| i 507 | 5'-AATGATACGGCGACCACCGAGATCTACAC**ACGTCCTG**GTTCAGAGTTCTACAGTCCGACGATC-3' | 10 |
| i 508 | 5'-AATGATACGGCGACCACCGAGATCTACAC**GTACTGAC**GTTCAGAGTTCTACAGTCCGACGATC-3' | 11 |
| i 701 | 5'-CAAGCAGAAGACGGCATACGAGAT**CGAGTAAT**GTGACTGGAGTTCAGACGTGTGCTCTTCCGATCT-3' | 12 |
| i 702 | 5'-CAAGCAGAAGACGGCATACGAGAT**TCTCCGGA**GTGACTGGAGTTCAGACGTGTGCTCTTCCGATCT-3' | 13 |
| i 703 | 5'-CAAGCAGAAGACGGCATACGAGAT**AATGAGCG**GTGACTGGAGTTCAGACGTGTGCTCTTCCGATCT-3' | 14 |
| i 704 | 5'-CAAGCAGAAGACGGCATACGAGAT**GGAATCTC**GTGACTGGAGTTCAGACGTGTGCTCTTCCGATCT-3' | 15 |
| i 705 | 5'-CAAGCAGAAGACGGCATACGAGAT**TTCTGAAT**GTGACTGGAGTTCAGACGTGTGCTCTTCCGATCT-3' | 16 |
| i 706 | 5'-CAAGCAGAAGACGGCATACGAGAT**ACGAATT**CGTGACTGGAGTTCAGACGTGTGCTCTTCCGATCT-3' | 17 |
| i 707 | 5'-CAAGCAGAAGACGGCATACGAGAT**AGCTTCAG**GTGACTGGAGTTCAGACGTGTGCTCTTCCGATCT-3' | 18 |
| i 708 | 5'-CAAGCAGAAGACGGCATACGAGAT**GCGCATTA**GTGACTGGAGTTCAGACGTGTGCTCTTCCGATCT-3' | 19 |
| i 709 | 5'-CAAGCAGAAGACGGCATACGAGAT**CATAGCCG**GTGACTGGAGTTCAGACGTGTGCTCTTCCGATCT-3' | 20 |
| i 710 | 5'-CAAGCAGAAGACGGCATACGAGAT**TTCGCGGA**GTGACTGGAGTTCAGACGTGTGCTCTTCCGATCT-3' | 21 |
| i 711 | 5'-CAAGCAGAAGACGGCATACGAGAT**GCGCGAGA**GTGACTGGAGTTCAGACGTGTGCTCTTCCGATCT-3' | 22 |
| i 712 | 5'-CAAGCAGAAGACGGCATACGAGAT**CTATCGCTG**TGACTGGAGTTCAGACGTGTGCTCTTCCGATCT-3' | 23 |

### NGS data analysis

cDOQ-related data retrieved from sequencing on Illumina platforms was first quality controlled using FastQC and reads afterwards trimmed with Trimmomatic to the first 40 nucleotides corresponding to the cDOQ hybridization sequence. Finally, trimmed reads were quantified using the alignment-free Sailfish tool under standard settings and a reference set including all cDOQ sequences. Analysis was performed using the European Galaxy Project platform (usegalaxy.eu).

RNAseq data was first quality controlled using FastQC and afterwards trimmed with Cutadapt, thus removing any remaining Illumina-related adapter sequences. Next, the trimmed reads were aligned to the reference sequences (either total tRNA references from Modomics and tRNAdb) using Bowtie 2, accounting for the high modification prevalence in tRNA by allowing one mismatch within a 22 nt sequence. Finally, mapped reads were analyzed in the Integrative Genomics Viewer.

### Example 1:

### Method design

For development of an accurate and sensitive high-throughput tRNA quantification method, the focus was placed on reduction of known major bias sources and a simple preparative approach, feasible with equipment available in most biochemical laboratories.

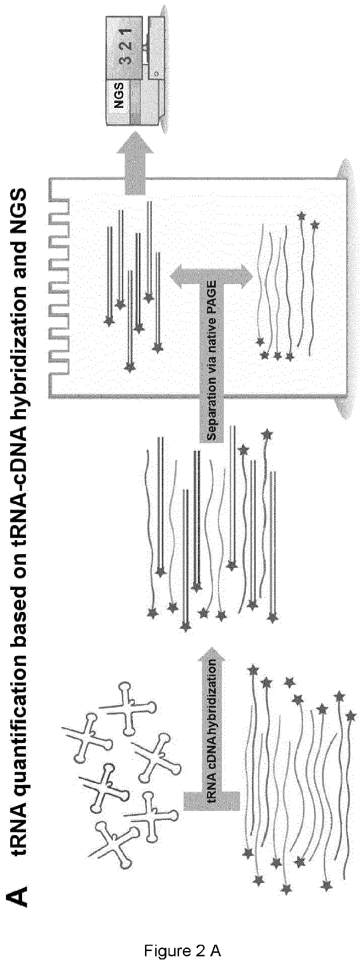

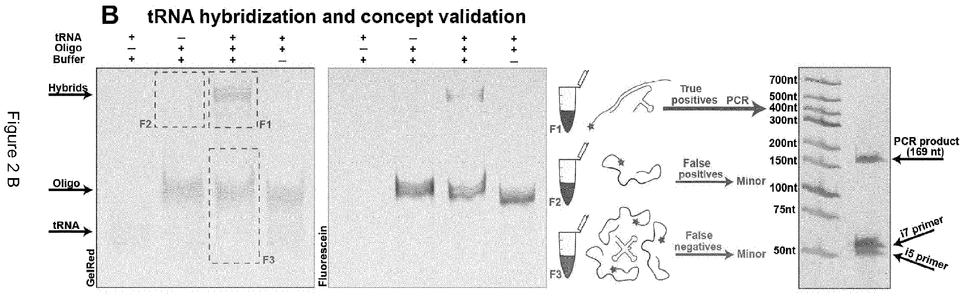

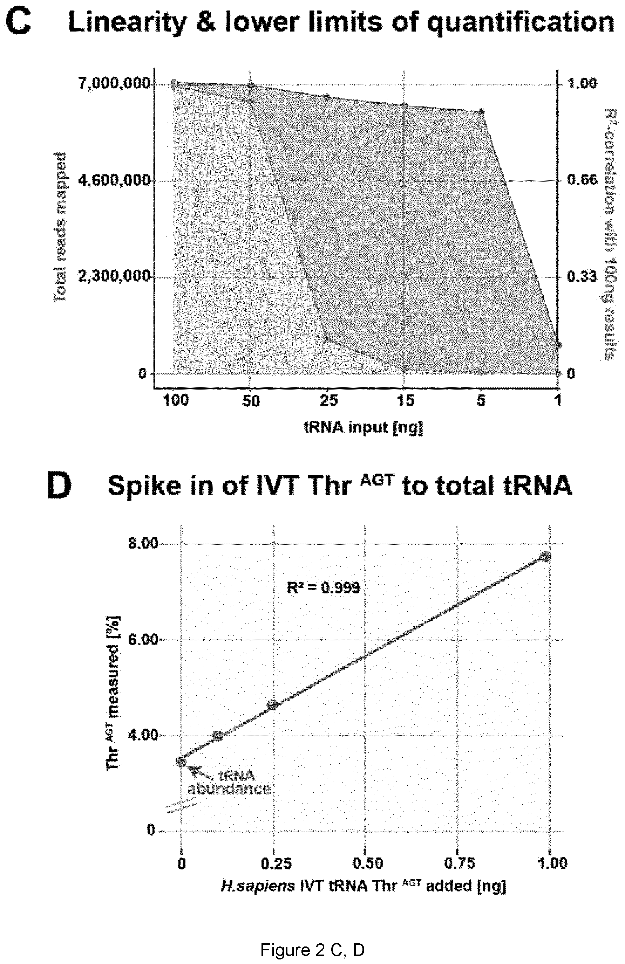

The core of the method of the present invention is a hybridization step of the tRNA of interest with an engineered cDNA. This step transmits quantitative information from tRNA to the resulting tRNA-cDNA duplexes in a 1:1 stoichiometry. The duplexes are then physically separated from non-hybridized cDNA, and the RNA is degraded. This leaves the quantitative information in single-stranded cDNA from which it is easily read out. In contrast to e.g. RNA sequencing methods, this foregoes the use of numerous enzymatic conversion steps such as ligation and reverse transcription, thereby avoiding the associated biases. The only remaining enzymatic step is an index PCR for the subsequent quantification by NGS sequencing (Fig. 2).

In practice, an organism-specific mix of some ~40 different cDNAs, each one corresponding to a major tRNA family, is hybridized to either total RNA or isolated total tRNA. Following denaturation and subsequent hybridization, the hybridization mixture is purified by native PAGE, thus physically separating tRNA-oligo hybrids from excess oligo and RNA contaminants. Upon elution from the native gel, a mixture of tRNA-oligo hybrids is obtained, in which the initial tRNA abundances are now represented by the abundance of each tRNA-specific oligo in the mixture. Quantification of the ~40 sequences was here developed for Illumina NGS system, but may conceivably be adapted to other analytical platforms. Conforming to this system, a bias-aware library preparation and analysis pipeline is applied developed as follows. The oligo-containing gel eluate is directly subjected to low-cycle index PCR to minimize amplification bias. The resulting full-length product is amenable to sequencing on all current Illumina platforms and straightforward analysis requiring a minimum of training or prior knowledge. Data treatment comprises quality control, trimming and alignment. The final output is a quantitative table in which the transcripts per million (TPM) represent the relative tRNA abundances in the initial RNA sample. Before application to several biological problems, this protocol was extensively scrutinized and validated with respect to various potential sources for errors and biases, as outlined below.

### Example 2:

### Method characterization and validation

### cDNA design integrates NGS adapters and tRNA hybridization sequences

Probe design for cDNA oligomers (abbreviated "cDOQ" for cDNA Oligo for quantification) addressed the following criteria, namely (i) facile detection and visualization, (ii) sequence elements for subsequent quantification and (iii) quantitative hybridization of the cognate tRNA. A combination of cDOQs subsequently called "cOQtail" needed to (iv) capture all known tRNA sequences of a given organism. Figure 5 A shows the general structure of cDOQs, comprising a 40 nt hybridization region complementary to the 3'end of a given tRNA isoacceptor family and framed by primer binding sites for the illumina i5 and i7 amplification sequences. A 5'-fluorescent dye aids visualization after PAGE.

### A surprising PCR amplification bias is minimal at 6 replication cycles

When the composition of an equimolar mixture of ~40 cDOQs, here directed against human tRNAs, was analyzed for relative content after 3, 6, 9, and 12 cycles of index PCR, a significant amplification bias was detected for 9 and 12 cycles. As shown in Figure 5 B, certain sequences were found enriched by 100% while others were decreased to 20% relative to the abundance at 2 cycles. This was unexpected given the identical primer binding sites, and was only weakly correlated to the GC content of the hybridizing sequences (Fig. 5 C). Interestingly, sequences behaved identically in three experimental replicates (executed on different days), demonstrating that the bias was indeed sequence specific and not random. Similar experiments on cDOQ mixtures covering tRNAs from other model organisms (Fig. 5 D) further confirmed this, and consistently showed minimal bias after 6 cycles, which was henceforth the default setting.

### cDOQ design is specific to single tRNA sequences

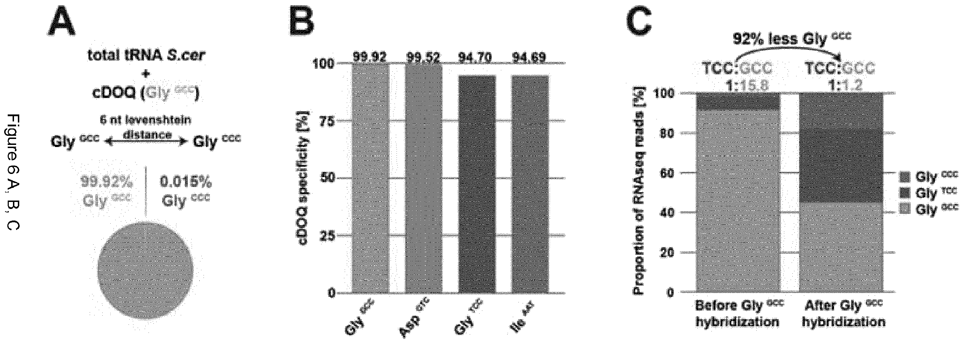

In an application to a first model organism, the 43 known cytosolic tRNA sequences of *Saccharomyces cerevisiae* were collapsed into 38 families, as known in the art. Within the corresponding 38 cDOQs, the shortest Levenshtein distance was 6 nucleotides, occurring between Gly ^{GCC} and Gly ^{CCC}. This corresponds to 15.0% of the hybridizing sequence, slightly exceeding the recommendations of 10 to 12.5% (4 to 5 nt). For experimental validation, 4 tRNAs were individually isolated from shifted bands in native gels after hybridization of total tRNA to their corresponding cDOQs. RNAseq of material isolated with the Gly ^{GCC}-cDOQ yielded 99% read alignment to the cognate tRNA Gly ^{GCC} family, and only 0.01% aligned to the nearest neighbor Gly^{CCC} (Fig. 6 A). Among the 4 tRNAs the average reads mapping to the cognate tRNA sequence was 97% and the minimum 95% (Fig. 6 B). Similar values were obtained with full-length cDOQs used for isolation of 7 different tRNAs. This establishes a Levenshtein distance of 6 nucleotides as efficient in the discrimination of similar sequences by 2 to 4 orders of magnitude. The cDOQ-Gly ^{GCC} also caused near complete depletion of the cognate tRNA from the remaining, *i.e.*, unshifted tRNA material (Fig. 6 C).

### Transfer of quantitative information from tRNA to cDOQs

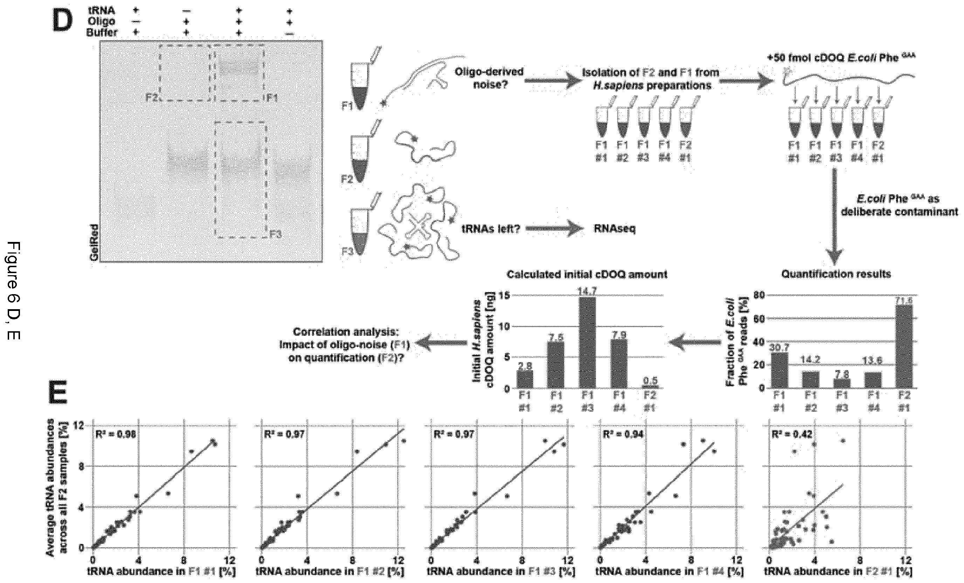

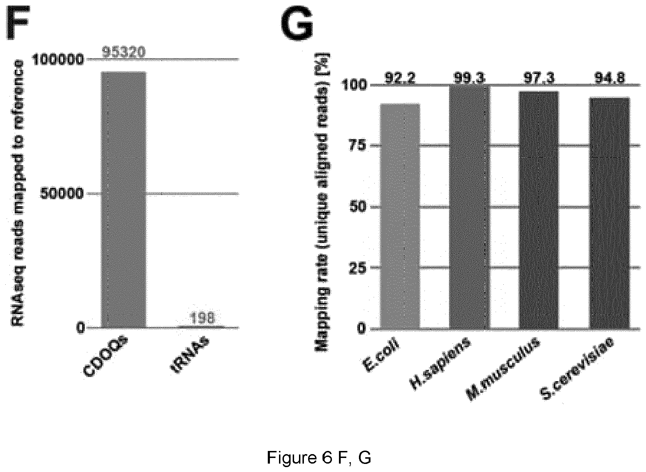

Moving from single sequences to total tRNA from *S*. *cerevisiae,* an analogous experiment was performed with a cocktail of 38 cDOQs. The mixture was heated and cooled to room temperature before separation on a native gel, which is shown in Figure 2 B. The left panel images all nucleic acids as stained by gel red, while the right panel displays only the fluorescein-labeled cDOQs. The resulting tRNA:cDOQ heteroduplexes in lane 3 were well separated due to their retardation relative to tRNAs and cDOQs as evident by comparison with lanes 1 and 2. The duplexes were excised from the region designated by a black rectangle, eluted, and further processed in the pipeline as "true positives" (F1, black sample). The gel region corresponding to duplexes in lane 2 (F2 blue: "no tRNA" false positive sample, blue rectangle in Figure 2 B) was similarly treated to determine signals that might have arisen from nonstandard migration of non-hybridized sDOQs. A detailed analysis shown in (Fig. 6 D, E) illustrated that the resulting signals in F2 were negligible. Finally, the bottom part of lane 3 was excised, and putative residual RNA submitted to an RNAseq protocol to potentially identify tRNAs that had failed to hybridize to the cDOQs (F3, green sample: "false negatives"). Its analysis, which is detailed in Example 3 (Fig. 6 F) showed only 0.2% of reads that could be mapped to cytosolic tRNA sequences, with the remainder corresponding to mitochondrial tRNA sequences.

It was thus concluded that all tRNA molecules were quantitatively hybridized and quantitative information on their composition contained in the cDOQs of the F1 black sample.

### Linearity to quantify the tRNA content of ~2000 cells

To determine the lower limits of the method of the present invention, a dilution series between 1 and 100 ng of *S. cerevisiae* tRNA was prepared and quantified. Figure 2 C shows the number of reads and their similarity to the 100 ng composition plotted for each amount of input tRNA. The ~7000 reads obtained from 1 ng of tRNA input would be sufficient, in theory, for relative quantification of the 38 species contained. However, the strong increase of coverage as well as similarity to the 100 ng sample at 5 ng and higher suggests an inherent limitation by noise. This places the lower limit for relative quantification at 5 ng or ~0.2 pmol tRNA. This amount corresponds to the content of ~ 30000 yeast cells, or the equivalent of 2000 mammalian cells.

For absolute quantification, a spike-in calibration with an *in vitro* transcript of human tRNA Thr ^{AGT} was quantified. The plot in Figure 2 D shows an R2 value of 0.99 that linearly correlates read numbers with 3 amounts of IVT in 25 ng each of total human tRNA. The value for zero spike corresponds to -34500 reads per million, equaling 0.86 ng of native tRNA Thr ^{AGT} contained in the total tRNA sample.

### Fast, precise, cost-effective, and high-throughput quantification of tRNA

In exploiting the full throughput-capability of NGS, multiplexing was established for parallel analysis of up to 96 samples on any of the current Illumina platforms. A typical run on a Nextseq 2000 platform with 96 samples input yielded over 4 million reads per sample, with an average Q-Score of 33.6. This thus implies less than one error in 2200 base calls, meaning that fewer than 2% of the 40nt recognition sequences was subject to a single base calling error mutation. Given the minimum Levenshtein distance of 6 between any two cDOQs, misassignments among cDOQs can be considered insignificant for quantification. Indeed and, in contrast to other methods, any multi-mapping was never observed. Typically, >98% of the reads mapped to the 38 yeast cDOQ sequences, providing excellent statistics way above the experimental limit established above (Figure 2 C). Of note, the data treatment is, in comparison to any other RNAseq approach, excessively simple and fast.

The turnaround time of such an analysis, starting from 96 samples of total RNA or total tRNA, is about five days, including 15 to 20 hours of hands-on time and 3 to 5 hours of data analysis, assuming the NGS is outsourced, in which case the cost including consumables is about 50 $ per sample. More importantly, this method evades all problems associated to reverse transcription, including the impact of RNA modifications, which are especially prevalent in tRNA. Consequently, so-called "jackpot" effects do not occur, and the relative distribution of tRNA families is much more physiologically plausible. Because the method easily lends itself to numerous problems in tRNA biology, species-specific sets of cDOQs were devised and validated for several model organisms including *E. coli,* mouse, and human, wherein mitochondrial tRNAs have also been included when appropriate (Tables 3 to 6). Among all organisms analyzed, the mapping rate ranged between 92 and 99 percent uniquely aligned reads (Fig. 6 G).

### Mitochondrial tRNA composition in mouse differs from human

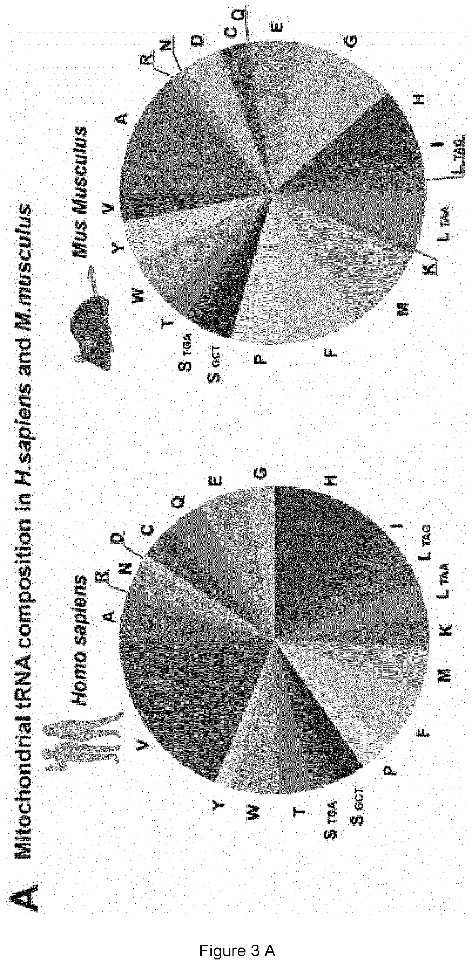

An analysis covering both cytosolic and mitochondrial tRNA compositions was conducted in several human cell lines and tissues (cf. below). The mitochondrial tRNA composition consistently showed a ~21% share of mt tRNA ^{Val} (Fig. 3 A), reflecting the substitution of 5S rRNA in mammalian mitochondria by this tRNA, which further validates both the method and its application to mammals. Interestingly, the analogous analysis of mouse tissues from brain and liver revealed only 3% abundance of mt-tRNA ^{Val}, while the most abundant mitochondrial species was now mt-tRNA ^{Ala}, featuring 13%, about 3x as much as in human. No literature on the identity of the 5S rRNA substitute in mouse could be found, and, significantly, a paper summarizing the findings in five mammalian species did not list mouse either. It is concluded that an experimental analysis likely failed, and the present data indicate that possibly the 5S rRNA role is filled by a mixture of different tRNA species in mouse.

### Gender-specific variation of mitochondrial tRNA abundances in Alzheimer patients

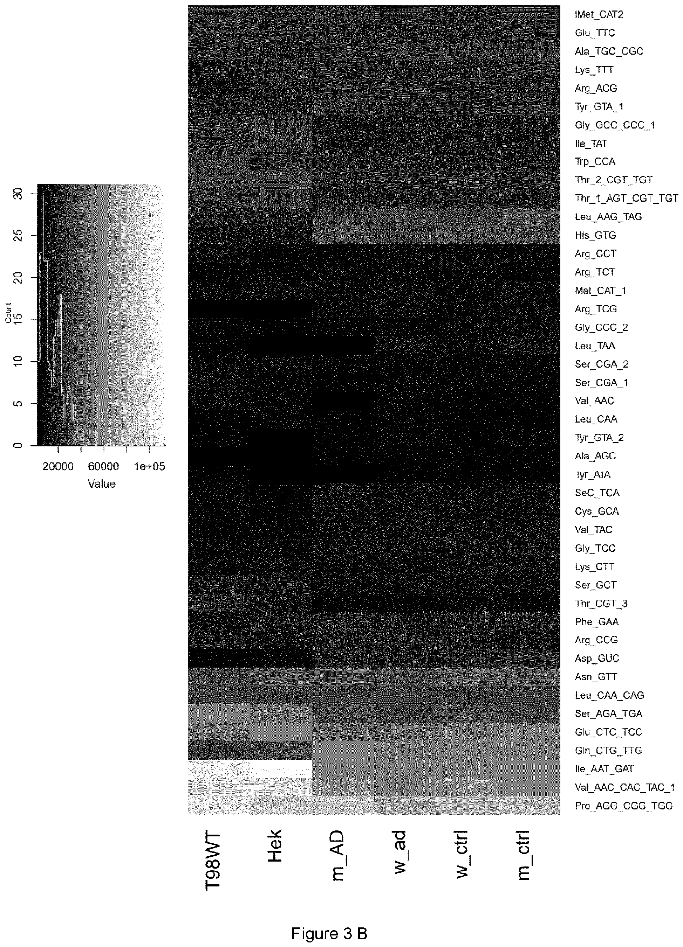

In the context of Alzheimer's disease (AD), the present high-throughput quantification approach was applied to a set of 25 human brain samples comprising 14 females and 11 males, both patients and healthy control. Additionally, several frequently used human cell lines were analyzed and compared to results from human tissue. Cytosolic tRNA abundance distributions showed no significant differences between patient groups, though clear differences when compared to human cell lines (Fig. 3 B). From 44 cDOQs targeting cytosolic tRNAs, 16 (HEK293) or 21 (T98G WT) showed significant differences compared to human cortex tissue.

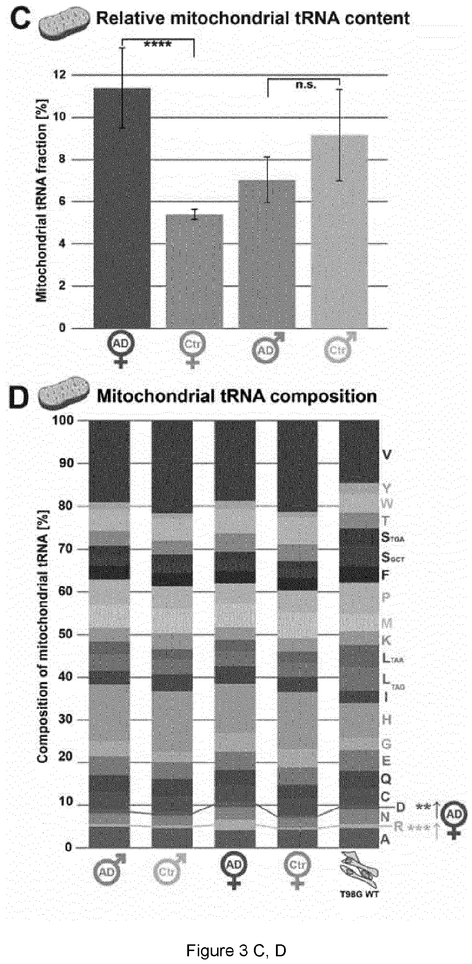

Strikingly, the overall abundance of mitochondrial tRNAs was significantly increased by 2-fold in women with AD, relative to the control group (Fig. 3 C). An increased level in male patients was not significant relative to the male control group. Investigating the relative abundances of individual mt-tRNAs, two significant differences could be observed for female patients. Both tRNA Arg ^{TCT} and Asp ^{GTC} displayed significantly increased abundance compared to all other patient groups (Fig. 3 D). Interestingly, with the exception of the female AD group, no significant differences could be observed between cell lines and human samples on a mitochondrial level.

The increase in mitochondrial tRNAs in female Alzheimer patients is unexpected, yet not implausible. Mitochondrial function is a central focus in Alzheimer research, and it is also known that the molecular pathogenesis differs significantly between female and male patients. This data is therefore considered an incentive to develop quantification tRNA in the AD field.

### Severe differences in tRNA pool composition of E. coli cytosol and polysomes are further impacted by paraquat treatment

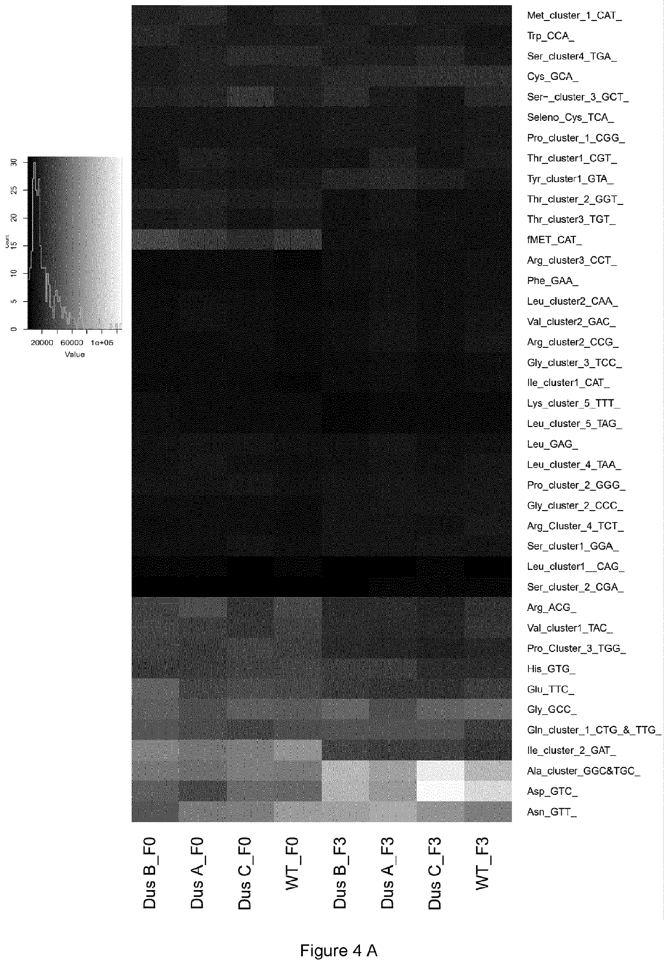

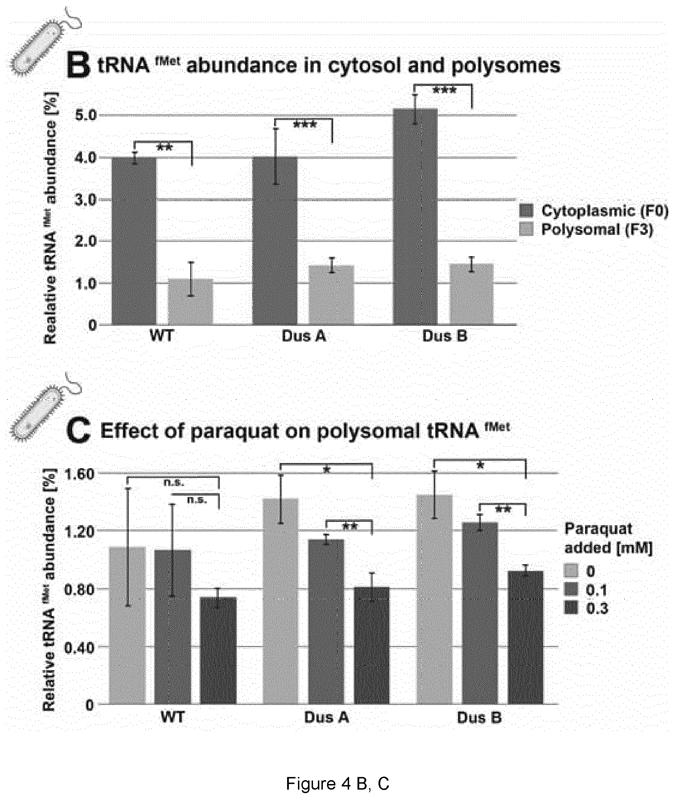

Exploiting the low input requirements, fractions of tRNAs isolated from actively translating polysomes (labelled F3 in Figure 4) were compared to the cytoplasmic pool of tRNAs (F0) in *E. coli.* As little as 21 ng of isolated tRNA from F3 fractions was sufficient to prepare technical triplicates. In addition to a wildtype *E. coli* K12 strain, the tRNA composition was compared in two knockout strains of tRNA modifications enzymes DusA and DusB. The respective enzymes catalyze dihydrouridine modifications in the namesake D-domain of tRNAs and might conceivably influence tRNA fitness and their usage on actively translating polysomes. A heatmap in Figure 4 gives a detailed view of the results. While tRNA abundances varied only slightly between different mutants, pronounced differences were observed between all F0 and their respective F3 fractions, with up to 18 tRNAs significantly under- or overrepresented (Fig. 4 A). These included tRNA ^{fMet} was far less abundant in all polysomal (F3) fractions compared to the cytosol with differences up to 3.5 fold (Fig. 4 B), which is highly plausible given that this tRNA is expected to occur only in freshly initiating ribosomes, *i.e.*, the 5'-most ribosome in each given polysome. F3 levels of tRNA ^{fMet} were similar across mutant strains with only minor differences between F0 fractions. Interestingly, the tRNA ^{fMet} populations in the F3 fractions decreased upon exposure to paraquat (Fig. 4 C). The latter was applied at sublethal concentrations to induce oxidative stress, leading to marked growth reduction, which is presumably related to the decreased population of initiator tRNA in translating polysomes.

### Example 3:

### Analysis of potential bias reveals only negligible impact of false negatives and false positives

Further characterization of the present method required analysis of potential noise derived from oligo secondary structures and the completeness of hybridization (Fig. 6 D). In order to determine potential noise, human tRNA-oligo hybrids (F1) of different human total RNA samples were excised from a native PAGE gel, as well as the corresponding height for samples only bearing oligo (F2), thus excising potential oligo running at hybrid-size. Following elution, 50 fmol of *E. coli* Phe ^{GAA} hybridization oligo were added to each sample as deliberate contaminant and internal standard. Following index PCR and Illumina sequencing, the percentage of reads mapped to *E. coli* Phe ^{GAA} (corresponding to 50 fmol oligo) was used to calculate the initial amount of *H. sapiens* hybridization oligos before PCR. Calculated oligo amounts ranged between 2.8 ng and 14.7 ng for hybrid (F1) fractions compared to 0.5 ng for the F2 fraction. Correlation analysis of retrieved data (Fig. 6 E) compared the individual measured tRNA abundances with the average tRNA abundances calculated from all hybrid (F1) fractions. Adjusted R²-values ranged between 0.94 and 0.98 for F1 fractions and 0.4 for the potential noise, thus indicating great differences in noise composition compared to hybrids. No dependency between R²-value and initial H. sapiens oligo amount could be observed as correlation was constantly high across all F1 fractions, implying a very limited impact of potential noise.

Furthermore, another fraction (F3) containing excess oligo and potentially unhybridized tRNAs was analyzed via RNAseq. Library preparation was based on the NEB next small RNAseq kit, using SuperScript IV as reverse transcriptase due to its improved sequence fidelity and processivity. Retrieved reads were processed and carefully aligned to the reference, considering potential mismatch induced by RNA modifications. Mapped reads comprised mostly oligo-derived sequences with only 0.2 % tRNA reads (Fig. 5 F), suggesting successful hybridization of the vast majority of tRNAs.

### Discussion and Outlook

Quantification methods for tRNA are a major driving force in tRNA biology, itself embedded in the larger field of protein translation. While tRNA biology has resurged along with RNA modification research, it has concomitantly become clear that tRNA modifications impose strong limitations to quantification. As a cardinal process in RNA quantification, cDNA hybridization to RNA can be designed to overcome negative effects from RNA modifications, typically by adjusting the cDNA length. Hence, when properly designed, cDNA based quantification approaches, such as northern blotting, OTTER, MST, mim-tRNAseq, AQRNAseq and RIP-chips, (cf. Table 7) can avoid biases, albeit at the expense of sequence information. As in the above methods, the approach taken in the present invention only reports information corresponding to the employed query sequences, thus missing modification information and details on isoacceptor composition within isoacceptor families covered by a common cDOQ. Among the previous, only chip-based methods tackle multiple sequences in a single experiment, which is otherwise the domain of deep sequencing approaches. However, RNAseq approaches have their intrinsic biases and statistical caveats in the quantification of complex mixtures, which are compounded in tRNA quantification, since modifications compound existing enzyme-based biases and introduce new ones. For example, reverse transcription is known to be hampered by RNA structure, which in turn is known to be reinforced by tRNA modifications. Reverse transcription arrest by modifications is known to affect quantification, albeit useful in the detection and quantification of those same modifications. Removal of a few selected modifications by enzymatic treatment e.g. with AlkB was introduced to improve cDNA synthesis and as a negative control to validate modification detection based on RT-arrest, yet introducing another enzymatic step prone to bias. Since, on a general level, incubation with any enzyme and the associate workup are not only time-consuming, put risk of additional RNA degradation and associated bias, a streamlined process was designed with a minimum of experimental steps and only a single enzymatic step. Ultimately, the enabling crucial step resides in the information transfer through hybridization of tRNAs with cDOQ, and subsequent separation. Consequently, strengths of the approach include very low material input requirements, low turnaround time, low cost, and low throughput. While the method only returns information on sequences that are queried by cDOQs, disregarding all other sequences improves the signal-to-noise and leads to extremely simple bioinformatics. The method is thus competitive or leading in any of the above categories (cf. Table 2), and its combined advantages make it possible to analyze complex experimental setups (typically 96 samples) with low abundant samples, e.g. tRNAs from polysomes or brain tissue in just one sequencing run. In summary, the method takes tRNA quantification to an advanced level of practical application to low input and high samples numbers. As demonstrated herein with AD biopsies, the method is clearly suited for analysis of clinical samples.

**Table 3: cDOQs designed for E. coli**

| **tRNA** | **Anticodon** | **Sequence** | **SEQ ID NO:** |
|---|---|---|---|
| Ala | GGC & TGC | 5'-AGACGTGTGCTCTTCCGATCTTGGTGGAGCTAAGCGGGATCGAACCGCAGACCTCCTGCATGATCGTCGGACTGTAGAACTCTGAAC-3' | 24 |
| Arg | ACG | 5'-AGACGTGTGCTCTTCCGATCTTGGTGCATCCGGGAGGATTCGAACCTCCGACCGCTCGGTTGATCGTCGGACTGTAGAACTCTGAAC-3' | 25 |
| Arg | CCG | 5'-AGACGTGTGCTCTTCCGATCTTGGCGCGCCCGACAGGATTCGAACCTGAGACCTCTGCCTCGATCGTCGGACTGTAGAACTCTGAAC-3' | 26 |
| Arg | CCT | 5'-AGACGTGTGCTCTTCCGATCTTGGTGTCCCCTGCAGGAATCGAACCTGCAATTAGCCCTTAGATCGTCGGACTGTAGAACTCTGAAC-3' | 27 |
| Arg | TCT | 5'-AGACGTGTGCTCTTCCGATCTTGGCGCGCCCTGCAGGATTCGAACCTGCGGCCCACGACTTGATCGTCGGACTGTAGAACTCTGAAC-3' | 28 |
| Asn | GTT | 5'-AGACGTGTGCTCTTCCGATCTTGGCTCCTCTGACTGGACTCGAACCAGTGACATACGGATTGATCGTCGGACTGTAGAACTCTGAAC-3' | 29 |
| Asp | GTC | 5'-AGACGTGTGCTCTTCCGATCTTGGCGGAACGGACGGGACTCGAACCCGCGACCCCCTGCGTGATCGTCGGACTGTAGAACTCTGAAC-3' | 30 |
| Cys | GCA | 5'-AGACGTGTGCTCTTCCGATCTTGGAGGCGCGTTCCGGAGTCGAACCGGACTAGACGGATTTGATCGTCGGACTGTAGAACTCTGAAC-3' | 31 |
| fMET | CAT | 5'-AGACGTGTGCTCTTCCGATCTTGGTTGCGGGGGCCGGATTTGAACCGACGACCTTCGGGTTGATCGTCGGACTGTAGAACTCTGAAC-3' | 32 |
| Gln | CTG & TTG | 5'-AGACGTGTGCTCTTCCGATCTTGGCTGGGGTACCTGGATTCGAACCTCGGAATGCCGGAATGATCGTCGGACTGTAGAACTCTGAAC-3' | 33 |
| Glu | TTC | 5'-AGACGTGTGCTCTTCCGATCTTGGCGTCCCCTAGGGGATTCGAACCCCTGTTACCGCCGTGGATCGTCGGACTGTAGAACTCTGAAC-3' | 34 |
| Gly | GCC | 5'-AGACGTGTGCTCTTCCGATCTTGGAGCGGGAAACGAGACTCGAACTCGCGACCCCGACCTTGATCGTCGGACTGTAGAACTCTGAAC-3' | 35 |
| Gly | CCC | 5'-AGACGTGTGCTCTTCCGATCTTGGAGCGGGCGAAGGGAATCGAACCCTCGTATAGAGCTTGGATCGTCGGACTGTAGAACTCTGAAC-3' | 36 |
| Gly | TCC | 5'-AGACGTGTGCTCTTCCGATCTTGGAGCGGGCAGCGGGAATCGAACCCGCATCATCAGCTTGGATCGTCGGACTGTAGAACTCTGAAC-3' | 37 |
| His | GTG | 5'-AGACGTGTGCTCTTCCGATCTTGGGGTGGCTAATGGGATTCGAACCCACGACAACTGGAATGATCGTCGGACTGTAGAACTCTGAAC-3' | 38 |
| Ile | GAT | 5'-AGACGTGTGCTCTTCCGATCTTGGTAGGCCTGAGTGGACTTGAACCACCGACCTCACCCTTGATCGTCGGACTGTAGAACTCTGAAC-3' | 39 |
| Ile | CAT | 5'-AGACGTGTGCTCTTCCGATCTTGGTGGCCCTTGCTGGACTTGAACCAGCGACCAAGCGATTGATCGTCGGACTGTAGAACTCTGAAC-3' | 40 |
| Leu | CAG | 5'-AGACGTGTGCTCTTCCGATCTTGGTGCGAGGGGGGGGACTTGAACCCCCACGTCCGTAAGGGATCGTCGGACTGTAGAACTCTGAAC-3' | 41 |
| Leu | CAA | 5'-AGACGTGTGCTCTTCCGATCTTGGTGCCGAAGGCCGGACTCGAACCGGCACGTATTTCTACGATCGTCGGACTGTAGAACTCTGAAC-3' | 42 |
| Leu | GAG | 5'-AGACGTGTGCTCTTCCGATCTTGGTACCGAGGACGGGACTTGAACCCGTAAGCCCTATTGGGATCGTCGGACTGTAGAACTCTGAAC-3' | 43 |
| Leu | TAA | 5'-AGACGTGTGCTCTTCCGATCTTGGTACCCGGAGCGGGACTTGAACCCGCACAGCGCGAACGGATCGTCGGACTGTAGAACTCTGAAC-3' | 44 |
| Leu | TAG | 5'-AGACGTGTGCTCTTCCGATCTTGGTGCGGGAGGCGAGACTTGAACTCGCACACCTTGCGGCGATCGTCGGACTGTAGAACTCTGAAC-3' | 45 |
| Lys | TTT | 5'-AGACGTGTGCTCTTCCGATCTTGGTGGGTCGTGCAGGATTCGAACCTGCGACCAATTGATTGATCGTCGGACTGTAGAACTCTGAAC-3' | 46 |
| Met | CAT | 5'-AGACGTGTGCTCTTCCGATCTTGGTGGCTACGACGGGATTCGAACCTGTGACCCCATCATTGATCGTCGGACTGTAGAACTCTGAAC-3' | 47 |
| Phe | GAA | 5'-AGACGTGTGCTCTTCCGATCTTGGTGCCCGGACTCGGAATCGAACCAAGGACACGGGGATTGATCGTCGGACTGTAGAACTCTGAAC-3' | 48 |
| Pro | CGG | 5'-AGACGTGTGCTCTTCCGATCTTGGTCGGTGATAGAGGATTCGAACCTCCGACCCCTTCGTCGATCGTCGGACTGTAGAACTCTGAAC-3' | 49 |
| Pro | GGG | 5'-AGACGTGTGCTCTTCCGATCTTGGTCGGCACGAGAGGATTTGAACCTCCGACCCCCGACACGATCGTCGGACTGTAGAACTCTGAAC-3' | 50 |
| Pro | TGG | 5'-AGACGTGTGCTCTTCCGATCTTGGTCGGCGAGAGAGGATTCGAACCTCCGACCCACTGGTCGATCGTCGGACTGTAGAACTCTGAAC-3' | 51 |
| Sec | TCA | 5'-AGACGTGTGCTCTTCCGATCTTGGCGGAAGATCACAGGAGTCGAACCTGCCCGGGACCGCTGATCGTCGGACTGTAGAACTCTGAAC-3' | 52 |
| Ser | GGA | 5'-AGACGTGTGCTCTTCCGATCTTGGCGGTGAGGGGGGGATTCGAACCCCCGATACGTTGCCGGATCGTCGGACTGTAGAACTCTGAAC-3' | 53 |
| Ser | CGA | 5'-AGACGTGTGCTCTTCCGATCTTGGCGGAGAGAGGGGGATTTGAACCCCCGGTAGAGTTGCCGATCGTCGGACTGTAGAACTCTGAAC-3' | 54 |
| Ser | GCT | 5'-AGACGTGTGCTCTTCCGATCTTGGCGGTGAGGCGGGGATTCGAACCCCGGATGCAGCTTTTGATCGTCGGACTGTAGAACTCTGAAC-3' | 55 |
| Ser | TGA | 5'-AGACGTGTGCTCTTCCGATCTTGGCGGAAGCGCAGAGATTCGAACTCTGGAACCCTTTCGGGATCGTCGGACTGTAGAACTCTGAAC-3' | 56 |
| Thr | CGT | 5'-AGACGTGTGCTCTTCCGATCTTGGTGCCGATAATAGGAGTCGAACCTACGACCTTCGCATTGATCGTCGGACTGTAGAACTCTGAAC-3' | 57 |
| Thr | GGT | 5'-AGACGTGTGCTCTTCCGATCTTGGTGCTGATACCCAGAGTCGAACTGCCGACCTCACCCTTGATCGTCGGACTGTAGAACTCTGAAC-3' | 58 |
| Thr | TGT | 5'-AGACGTGTGCTCTTCCGATCTTGGTGCCGACTACCGGAATCGAACTGGTGACCTACTGATTGATCGTCGGACTGTAGAACTCTGAAC-3' | 59 |
| Trp | CCA | 5'-AGACGTGTGCTCTTCCGATCTTGGCAGGGGCGGAGAGACTCGAACTCCCAACACCCGGTTTGATCGTCGGACTGTAGAACTCTGAAC-3' | 60 |
| Tyr | GTA | 5'-AGACGTGTGCTCTTCCGATCTTGGTGGTGGGGGAAGGATTCGAACCTTCGAAGTCGATGACGATCGTCGGACTGTAGAACTCTGAAC-3' | 61 |
| Val | TAC | 5'-AGACGTGTGCTCTTCCGATCTTGGTGGGTGATGACGGGATCGAACCGCCGACCCCCTCCTTGATCGTCGGACTGTAGAACTCTGAAC-3' | 62 |
| Val | GAC | 5'-AGACGTGTGCTCTTCCGATCTTGGTGCGTCCGAGTGGACTCGAACCAACGACCCCCACCATGATCGTCGGACTGTAGAACTCTGAAC-3' | 63 |

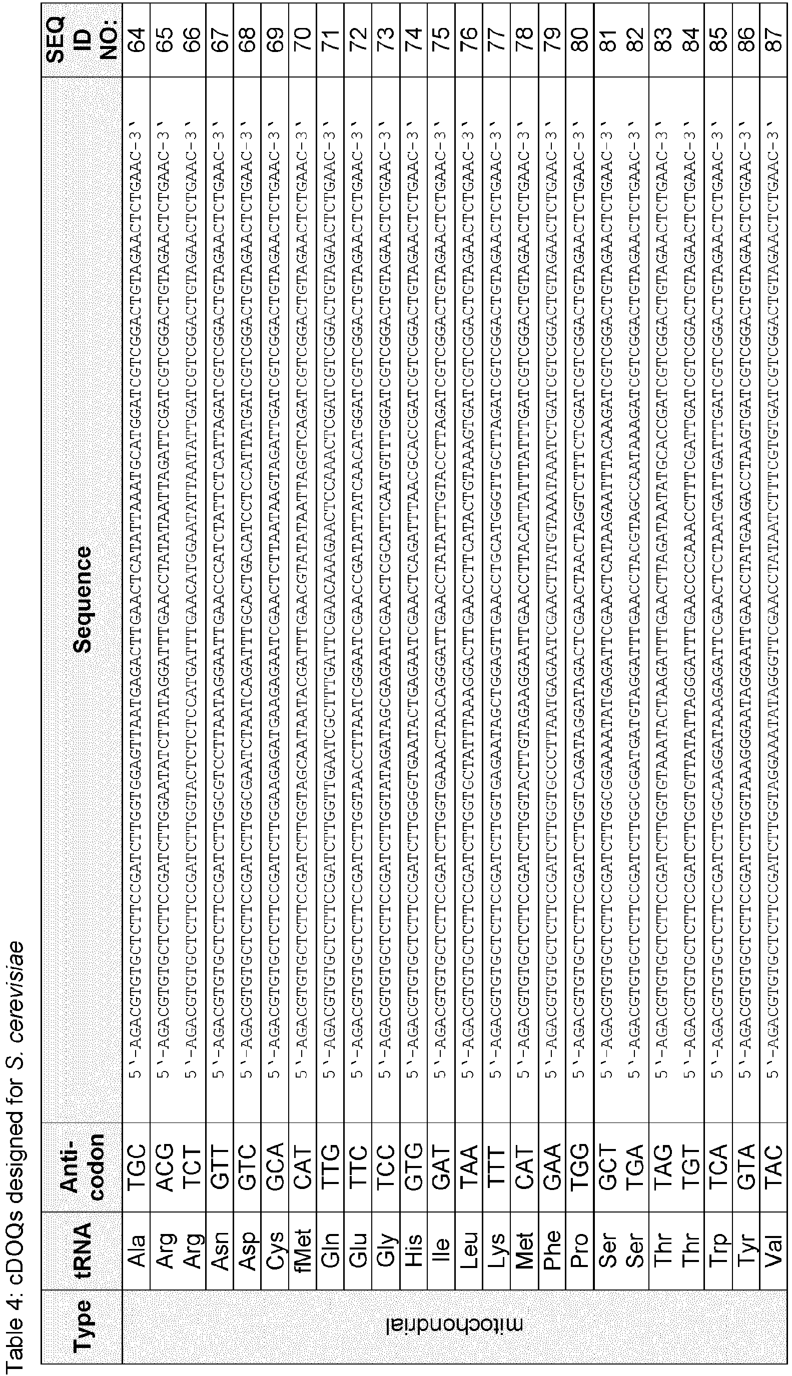

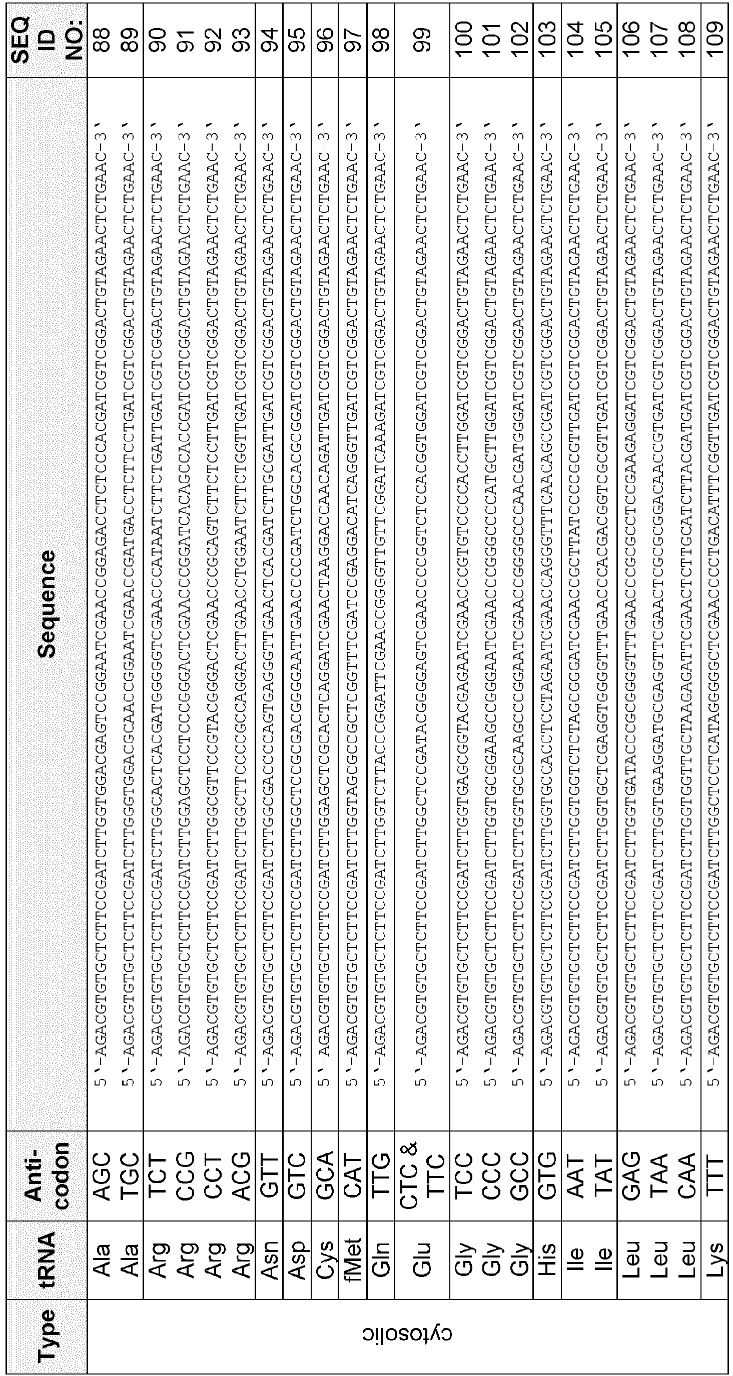

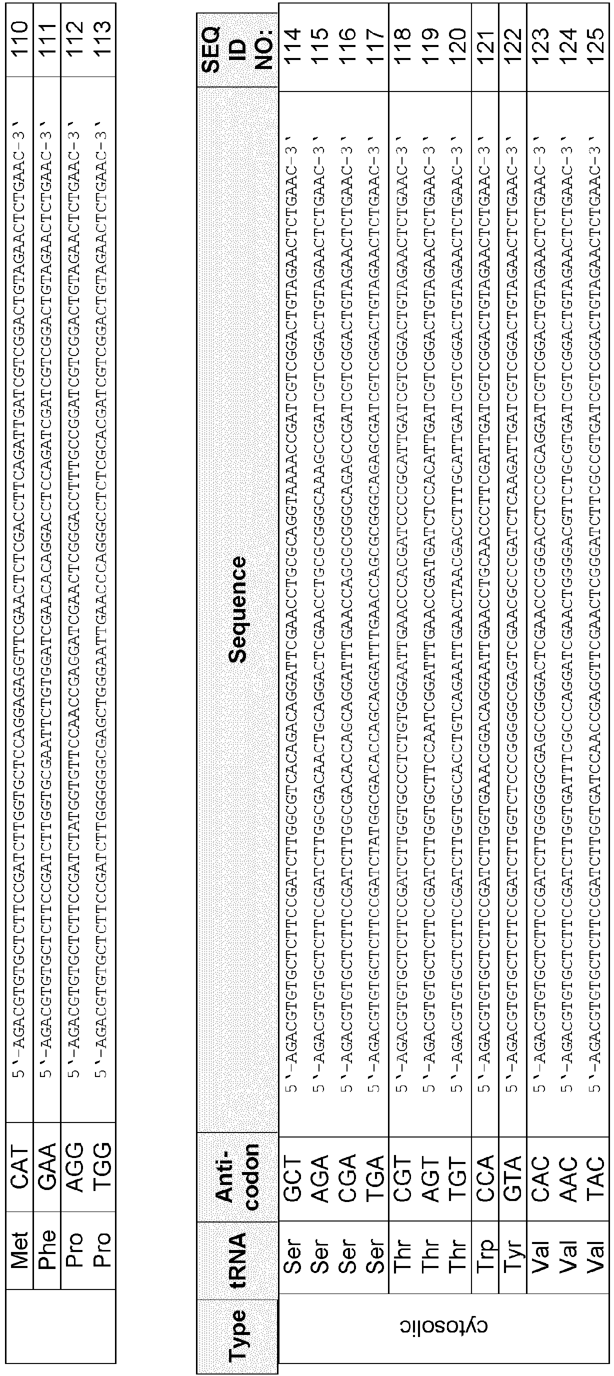

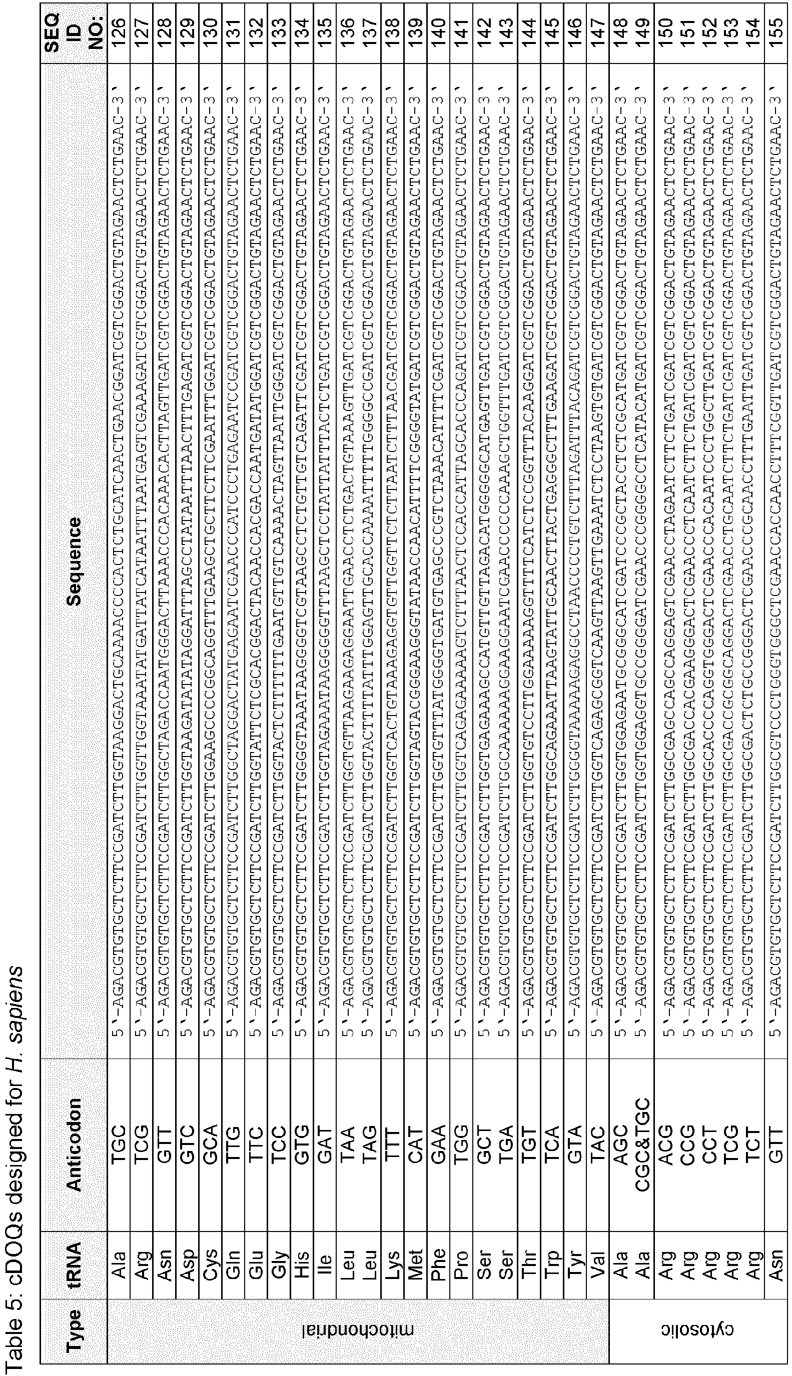

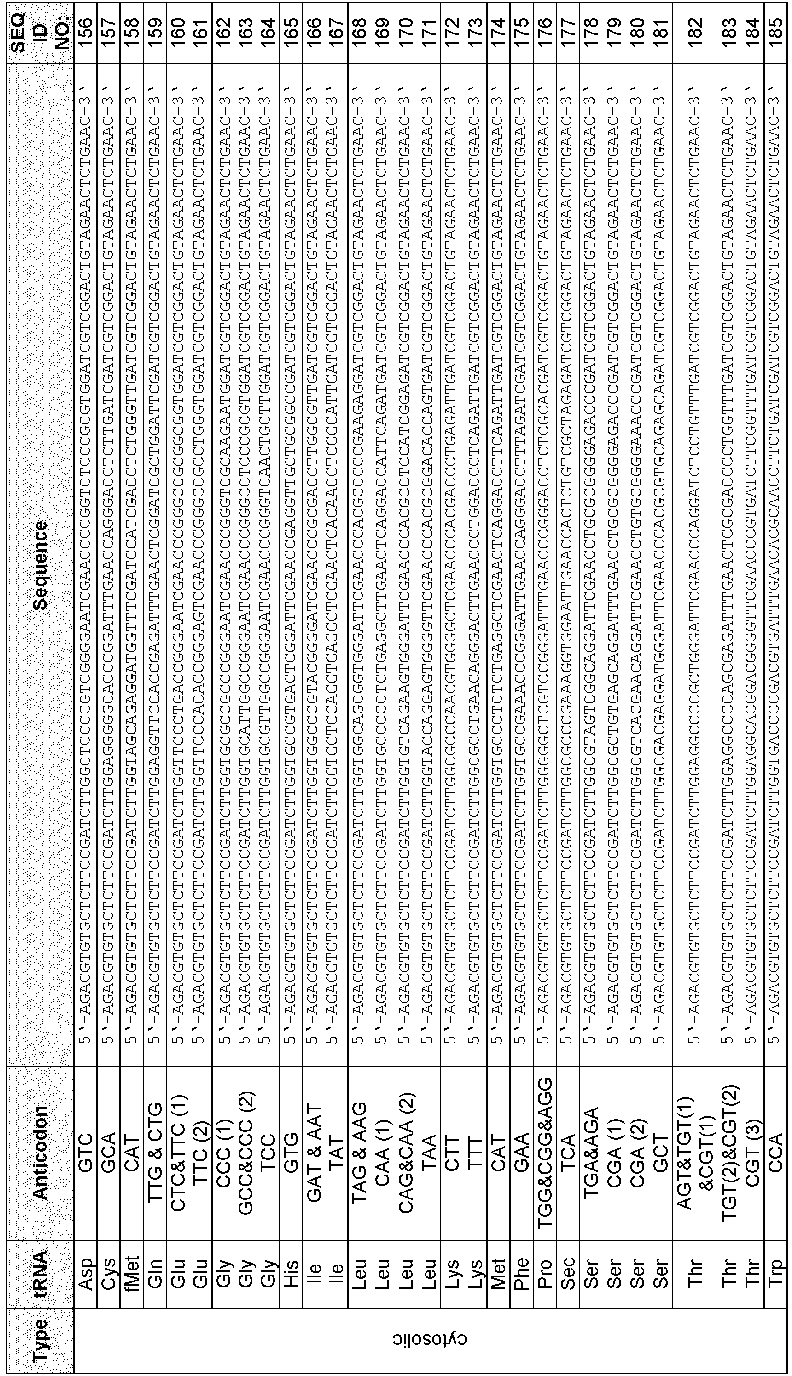

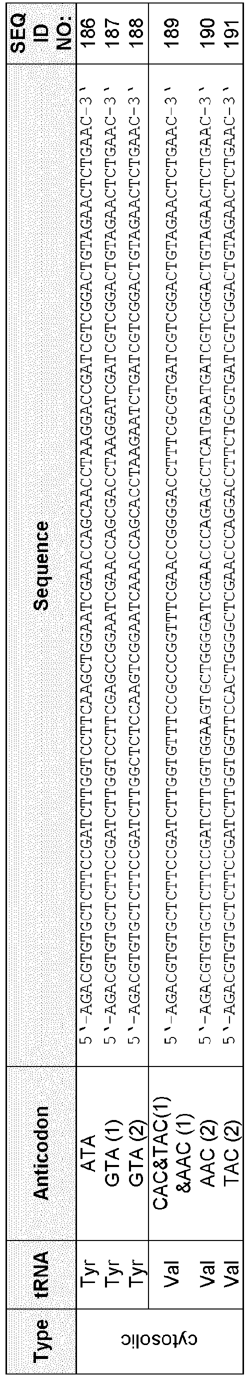

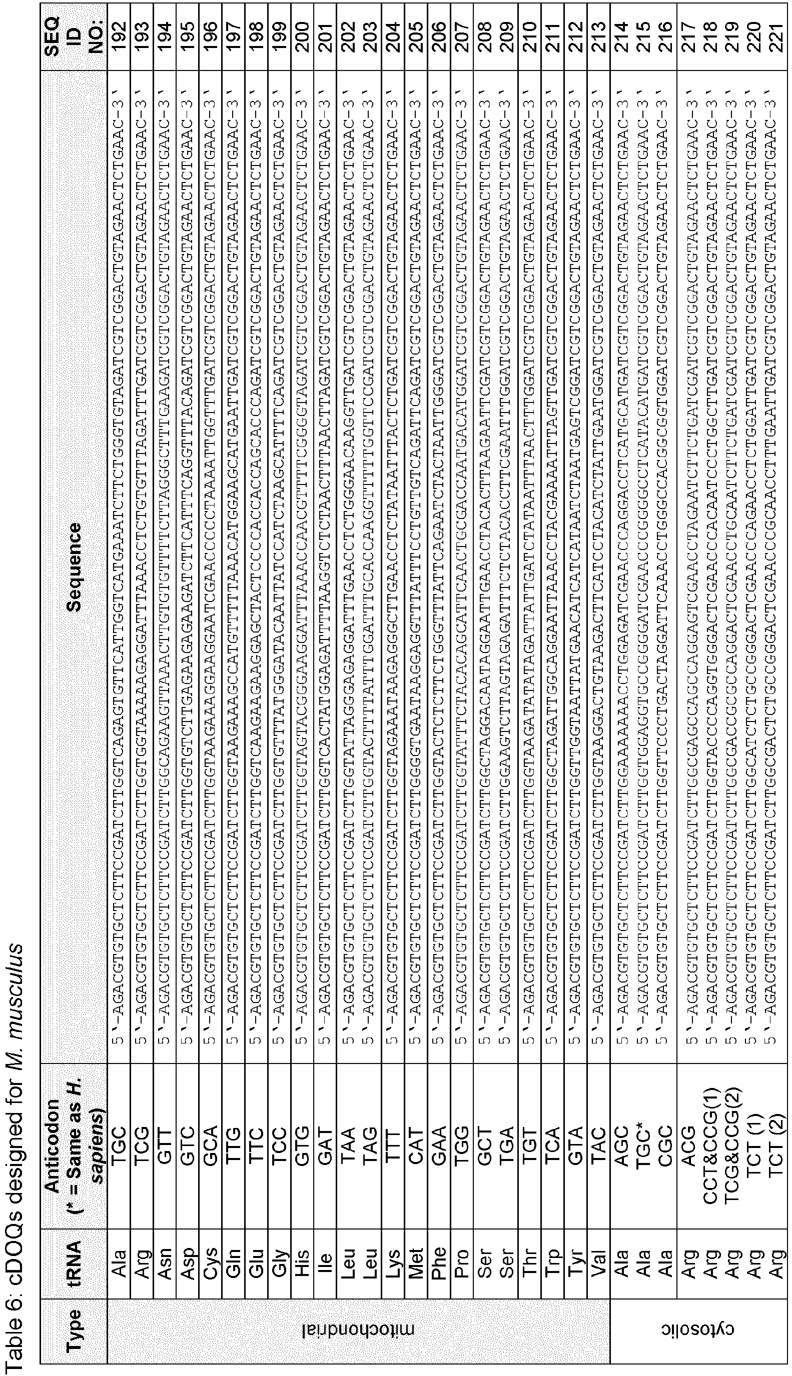

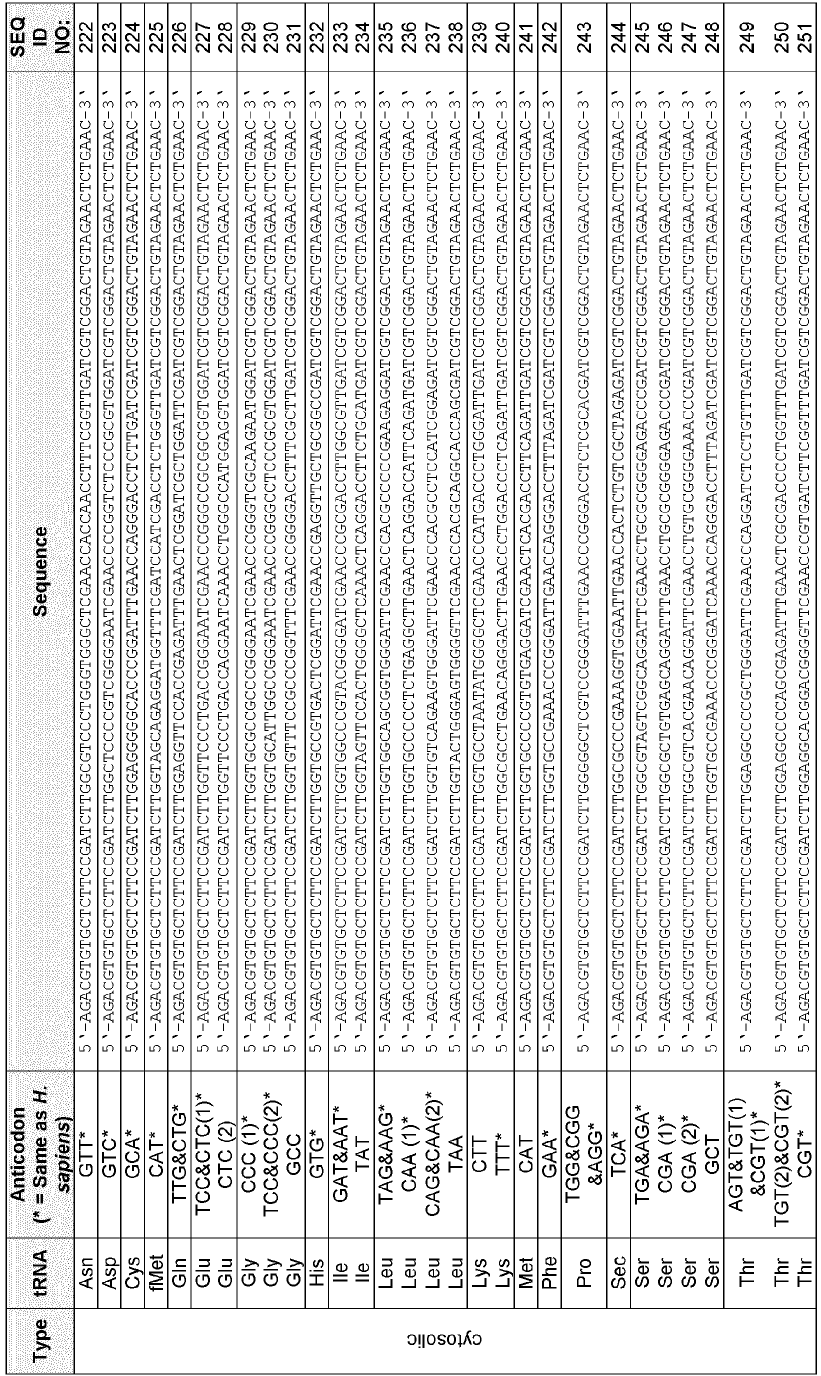

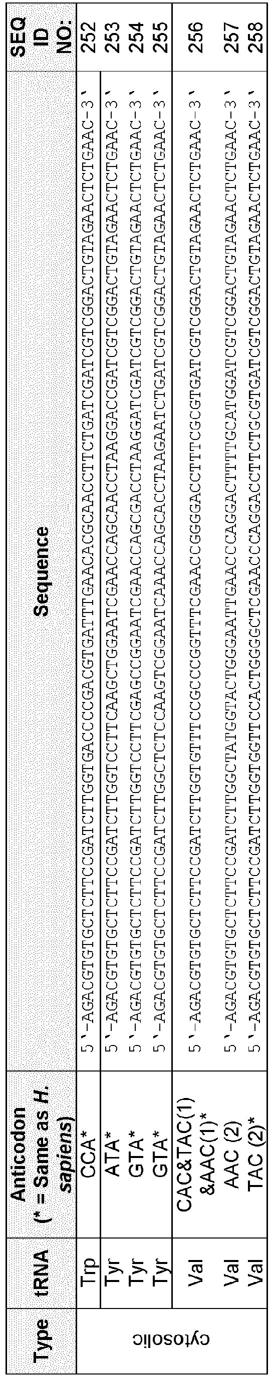

**Table 7: Comparative table of exemplary tRNA quantification methods**

| **Name, if applicable** | **Method** | **Amount** | **Parallel quantification of all tRNAs?** | **Enzymatic steps** |
|---|---|---|---|---|
| mim-tRNAseq | RNAseq | 50-200 ng total tRNA | Yes | 6 |
| OTTER | In gel fluorescence | 2 µg total RNA | No | 1 |
| AQRNAseq | RNAseq | 50 ng total tRNA | Yes | 10 |
| QuantM-tRNA-seq | RNAseq | 1 µg deacetyl. total RNA | Yes | 5 |
| MST | Microscale Thermophoresis | 1-2 µg tot tRNA | No | 0 |
| tRNA microarray | Microarray | 1-2 µg fluoro labeled tRNA | No | 1 |
| Hydro-tRNAseq | RNAseq | 20 µg total RNA | Yes | 6 |
| YAMAT-seq | RNAseq | 1 µg deac. total RNA | Yes | 3 |
| DM-TGIRT-seq | RNAseq + alkB | 100 ng demethyl. tRNA or 1 µg tot. RNA | Yes | 4 |
| ARM-seq | RNAseq + alkB | 1 µg total RNA | Yes | 7 |
| tRNA-seq | RNAseq | - | Yes | 6 |
| tRNA-microarrays | Microarray | 1 µg fluoro labeled total RNA | No | 1 |
| - | 2D gel radiolabel | 5 µg total RNA | No | 0 |
| - | 2D gel radiolabel | - | No | 0 |
| Method of the present invention | Hybridization + NGS | 5-25 ng total tRNA | Yes | 1 |

## Claims

1. A method for the quantification of one or more tRNA species in a tissue sample or cell sample derived from a specific organism, comprising the steps of:
(a) providing a tissue sample or cell sample derived from a specific organism;
(b) extracting total RNA from said tissue sample or cell sample;
(c) adding one or a mixture of more than one tRNA-complementary DNA oligonucleotide(s) to the total RNA obtained in step (b),
wherein each of said tRNA-complementary DNA oligonucleotides contains a nucleotide sequence that specifically hybridizes to one specific tRNA of the organism from which the tissue or cell sample is derived, or to one specific tRNA family of said organism;
(d) allowing for hybridization of the tRNA-complementary DNA oligonucleotides added in step (c) to the tRNAs, thereby forming tRNA-tRNA-complementary DNA oligonucleotide heteroduplexes;
(e) performing native polyacrylamide gel electrophoresis with the mixture obtained in step (c) after hybridization in step (d), thereby separating tRNA-tRNA-complementary DNA oligonucleotide heteroduplexes, non-hybridized tRNA-complementary DNA oligonucleotides, and other RNA species from each other;
(f) purifying the tRNA-tRNA-complementary DNA oligonucleotide heteroduplexes from the native polyacrylamide gel;
(g) performing an index polymerase chain reaction (index PCR) on the tRNA-tRNA-complementary DNA oligonucleotide heteroduplexes obtained in step (f) using suitable index primers, thereby separating the tRNA-complementary DNA oligonucleotides from the tRNAs;
(h) purifying the PCR product obtained in step (g);
(i) subjecting the purified PCR product obtained in step (h) to NGS; and
(j) counting the number of times each tRNA-complementary DNA oligonucleotide sequence is found in the sequencing data obtained in step (i), wherein said number directly corresponds to the abundance of the respective tRNA species in the cell sample or tissue sample.

2. The method of claim 1, wherein the hybridization region of the tRNA-complementary DNA oligonucleotides comprises about 40 nucleotides.

3. The method of claim 1 or claim 2, wherein the hybridization region of the tRNA-complementary DNA oligonucleotides can hybridize to the 3'-end of the tRNAs, or to the 5'-end of the tRNAs.

4. The method of any one of claims 1 to 3, wherein the tRNA-complementary DNA oligonucleotides added in step (c) contain nucleotide sequences that are required for further sequencing on a next generation sequencing (NGS) platform up- and downstream of said nucleotide sequence that specifically hybridizes to one specific tRNA of the organism from which the tissue or cell sample is derived.

5. The method of any one of claims 1 to 4, wherein the tRNA-complementary DNA oligonucleotides added in step (c) contain a fluorescent tag or biotin.

6. The methods of any one of claims 1 to 5, wherein in step (c) a mixture of tRNA-complementary oligonucleotides for each tRNA species of the specific organism is added to the total RNA obtained in step (b).

7. The method of any one of claims 1 to 6, wherein step (d) comprises denaturing of the solution containing the tRNAs and the tRNA-complementary DNA oligonucleotides and subsequent gradual cooling of said solution.

8. The method of any one of claims 1 to 7, wherein purification of the tRNA-tRNA-complementary DNA oligonucleotide heteroduplexes from the native polyacrylamide gel in step (f) comprises excision of the respective band(s) from the native polyacrylamide gel and elution of the tRNA-tRNA-complementary DNA oligonucleotide heteroduplexes from the excised gel fragment(s).

9. The method of any one of claims 1 to 8, wherein the PCR in step (g) is performed for 2 to 6 PCR cycles.

10. The method of any one of claims 1 to 9, wherein during the index PCR in step (g), further nucleotide sequences that are required for further sequencing on an NGS platform are added to said tRNA-complementary DNA oligonucleotides.

11. The method of any one of claims 1 to 10, wherein the nucleotide sequences that are required for further sequencing on a next generation sequencing (NGS) platform, present in the tRNA-complementary DNA oligonucleotides added in step (c) and/or added during the index PCR in step (g), are primer binding sites.

12. The method of any one of claims 1 to 11, wherein every tRNA species of the specific organism is quantified.

13. The method of any one of claims 1 to 12, wherein the tissue sample or cell sample contains (i) 50 ng or less total tRNA, and/or (ii) 2 pmol or less total tRNA.

14. The method of any one of claims 1 to 13, wherein the tissue sample or cell sample is derived from *Homo sapiens, Mus musculus, Saccharomyces cerevisiae, Drosophila* melanogaster, *Caenorhabditis elegans,* or *Escherichia coli.*

15. The method of claim 14, wherein
(i) the tissue sample or cell sample is derived from *Homo sapiens,* and the one or more than one tRNA-complementary DNA oligonucleotide(s) are selected from the group consisting of oligonucleotides having the nucleotide sequences of SEQ ID NOs: 126 to 191;
(ii) the tissue sample or cell sample is derived from *Mus musculus,* and the one or more than one tRNA-complementary DNA oligonucleotide(s) are selected from the group consisting of oligonucleotides having the nucleotide sequences of SEQ ID NOs: 192 to 258;
(iii) the cell sample is derived from *Saccharomyces cerevisiae,* and the one or more than one tRNA-complementary DNA oligonucleotide(s) are selected from the group consisting of oligonucleotides having the nucleotide sequences of SEQ ID NOs: 64 to 125; or
(iv) the cell sample is derived from *Escherichia coli,* and the one or more than one tRNA-complementary DNA oligonucleotide(s) are selected from the group consisting of oligonucleotides having the nucleotide sequences of SEQ ID NOs: 24 to 63.
